# EUROPÄISCHE PATENTSCHRIFT

(11) **EP 4 617 635 B1**
(45) Veröffentlichungstag und Bekanntmachung des Hinweises auf die Patenterteilung: **22.04.2026**
(21) Anmeldenummer: 25162883.0
(22) Anmeldetag: 11.03.2025
(51) Int. Cl.: G01M 3/32, A61J 1/00, B65D 90/50, A61J 1/06, A61J 1/14, A61M 1/16

(54) **PRÜFVORRICHTUNG UND VERFAHREN ZUR DICHTIGKEITSPRÜFUNG EINES MEDIZINISCHEN BEHÄLTERS, SOWIE PRÜFSYSTEM MIT PRÜFVORRICHTUNG UND MEDIZINISCHEM BEHÄLTER**
TESTING DEVICE AND METHOD FOR TESTING THE TIGHTNESS OF A MEDICAL CONTAINER, AND TESTING SYSTEM COMPRISING TESTING DEVICE AND MEDICAL CONTAINER
DISPOSITIF DE CONTRÔLE ET PROCÉDÉ DE CONTRÔLE D'ÉTANCHÉITÉ D'UN RÉCIPIENT MÉDICAL, ET SYSTÈME DE CONTRÔLE DOTÉ DU DISPOSITIF DE CONTRÔLE ET RÉCIPIENT MÉDICAL

(30) Priorität: 13.03.2024 DE 102024107162
(43) Veröffentlichungstag der Anmeldung: 17.09.2025
(73) Patentinhaber: B. Braun Avitum AG, 34212 Melsungen (DE)
(72) Erfinder: LAMPE, Michael, 49770 Herzlake (DE)
(74) Vertreter: Winter, Brandl - Partnerschaft mbB

(56) Entgegenhaltungen:
- DE-A1- 102017 204 102
- US-A- 4 459 843
- US-A- 4 715 214
- US-A1- 2014 083 169

## Beschreibung

### Technisches Gebiet

Die vorliegende Offenbarung betrifft eine Prüfvorrichtung zur Dichtigkeitsprüfung eines medizinischen Behälters, ein Prüfsystem mit der Prüfvorrichtung und dem zu prüfenden, medizinischen Behälter, sowie ein Verfahren zur Dichtigkeitsprüfung eines medizinischen Behälters.

### Technischer Hintergrund

Gattungsgemäße, medizinische Behälter beinhalten medizinische, insbesondere pulverförmige und/oder flüssige chemische Substanzen, wie beispielsweise Trockenkonzentrat, welches etwa Salze und eine Säure, z.B. Essigsäure oder Zitronensäure, und potenziell auch andere Substanzen, z.B. Glukose, enthält.

Auf die Anmelderin gehen als Kartuschen oder Fässer ausgestaltete Behälter zurück, die beispielsweise mehrere hundert Liter Fassungsvermögen aufweisen. Insbesondere dienen sie der Lagerung und dem Transport von Trockenkonzentrat, beispielsweise zur Herstellung einer für die Dialyse benötigten Dialysierflüssigkeit. Die Anlieferung von trockenem anstatt flüssigem Konzentrat in eine medizinische Dialyseeinrichtung und die erst dort erfolgende Herstellung/ das dortige In-Lösung-Bringen, stellen eine erhebliche Reduktion an Transportgewicht und Transportkosten dar und verbessern die Energiebilanz der Dialyse erheblich.

In der Dialyseeinrichtung wird das Trockenkonzentrat innerhalb der Kartusche in Lösung gebracht. Da dieser Produktionsschritt zur Herstellung der Lösung nicht in einer herkömmlichen, technischen Produktion erfolgt, sondern in der medizinischen Einrichtung, und weil eine Leckage des Behälters in der medizinischen Einrichtung ein hohes Kosten- und Sicherheitsrisiko darstellt, ist es von hoher Wichtigkeit, die Dichtigkeit der Behälter/Kartuschen zu gewährleisten.

Hierzu wird jeder Behälter einer Dichtigkeits- oder Leckageprüfung unterzogen.

Gemäß einer üblichen Vorgehensweise werden die noch unbefüllten Behälter mit Wasser befüllt und durchspült. Im Anschluss wird von einem Bedienpersonal eine Sichtkontrolle durchgeführt und es erfolgt die Kontrolle auf feuchte Stellen oder einen Austritt von Lösung. Die Qualität einer derartigen Kontrolle ist individuell, das heißt, sie hängt in hohem Maße von der Erfahrung des Bedienpersonals ab. Das Befüllen eines mehrere hundert Liter fassenden Behälters, sowie das im Anschluss an die Prüfung notwendige Leersaugen und Trocknen nehmen zudem jeweils mehrere Minuten in Anspruch und stellen einen zeit- und kostenintensiven Prozessschritt dar. Die Verwendung von Wasser als Prüfmittel hat darüber hinaus den Nachteil, dass bereits befüllte Behälter, das heißt solche, in denen bereits Trockensubstanz oder -konzentrat enthalten ist, nicht geprüft werden können, ohne dabei das Trockenkonzentrat in Lösung zu bringen. Besteht Unsicherheit, ob der bereits mit Trockenkonzentrat befüllte Behälter ausreichend dicht ist, muss er daher erneut entleert werden, bevor er geprüft werden kann. Das ist aufwendig und birgt das Risiko, dabei das bereits im Behälter befindliche Trockenkonzentrat aber auch die Umgebung zu kontaminieren.

Die US 2014 0 083 169 A1 beschreibt eine tragbare Testvorrichtung und ein Verfahren zur Prüfung der Integrität von flexiblen Behältern, insbesondere von aseptischen flexiblen Folienbeuteln. Die Behälter werden mit einem sterilen Gas aufgeblasen, um Falten zu entfernen und einen Drucksollwert festzulegen. Der Druckabfall wird über einen bestimmten Zeitraum gemessen. Wenn der Druckverlust einen bestimmten Schwellenwert nicht überschreitet, wird die Integrität des Beutels bestätigt. Die Vorrichtung umfasst eine Steuerung, einen Druckwandler, eine Stromquelle, ein Gebläse und eine Schnittstelle. Das Verfahren und die Vorrichtung vermeiden Heliumtests, verwenden stattdessen Luft und reduzieren Faltenbildung vor der Befüllung.

Die US 4 459 843 A beschreibt eine Vorrichtung und ein Verfahren zum Testen von Behältern auf Druckverlust. Die Vorrichtung misst die Druckdifferenz in einem Behälter über die Zeit und vergleicht sie mit einem akzeptablen Wert. Ein Computer passt diesen Wert automatisch an.

### Zusammenfassung der vorliegenden Offenbarung

Die Aufgabe der vorliegenden Offenbarung ist demgegenüber, die Nachteile aus dem Stand der Technik zu vermeiden oder zumindest zu mindern und insbesondere eine Prüfvorrichtung zur Dichtigkeitsprüfung eines medizinischen Behälters, ein Prüfsystem aus Prüfvorrichtung und zu prüfendem Behälter, sowie ein Verfahren zur Dichtigkeitsprüfung des medizinischen Behälters zur Verfügung zu stellen, welche bei gleicher Zuverlässigkeit des Prüfergebnisses einen geringeren Aufwand darstellen.

Die Aufgabe wird hinsichtlich einer Prüfvorrichtung zur Dichtigkeitsprüfung eines medizinischen Behälters offenbarungsgemäß durch die Merkmale des Anspruchs 1 gelöst, hinsichtlich des Prüfsystems durch die Merkmale des Anspruchs 12 und hinsichtlich des Verfahrens offenbarungsgemäß durch die Merkmale des Anspruchs 13 gelöst. Vorteilhafte Ausführungsformen sind in den Unteransprüchen beansprucht und werden insbesondere nachfolgend erläutert.

Ein Grundgedanke der vorliegenden Offenbarung sieht vor, die Dichtigkeit des Behälters trocken durchzuführen, wobei anstatt einer Flüssigkeit ein Gas, insbesondere Druckluft, als Prüf-Fluid vorgesehen ist. Die trockene Prüfung bietet dabei die Vorteile, dass der Behälter im Nachgang nicht getrocknet werden muss, und dass die Prüfung selbst bei befülltem Container erfolgen kann, da eine im Behälter befindliche Substanz oder Trockensubstanz nicht - anders als bei Wasser als Prüf-Fluid - in Lösung gebracht wird. Im Falle der Prüfung eines mit oxidationsfähiger Trockensubstanz befüllten Behälters kann ein inertes Gas als Prüf-Fluid Verwendung finden.

Gegenstand der Erfindung sind eine Prüfvorrichtung, ein Prüfsystem und ein Verfahren, mit dem die trockene Prüfung mittels einer Druckprüfung erfolgt, indem ein medizinischer Behälter, insbesondere eine Trockenkonzentrat-Kartusche, mit Druckluft beaufschlagt wird und der Druck erfasst wird. Der zeitabhängig erfasste Druck wird mit einer hinterlegten, zeitabhängigen Druckkennlinie abgeglichen. Erfasste Druckwerte, die außerhalb einer vorbestimmten Toleranz zur Druckkennlinie liegen, deuten auf Leckagen oder Risse an Verbindungsstellen, Schweißnähten oder am Behälterkörper hin. Das Prüfergebnis wird einem Anwender gemeldet.

Der Behälter ist vorgesehen und ausgestaltet, dass in ihm eine medizinische Trockensubstanz oder ein medizinisches Trockenkonzentrat in Lösung gebracht wird. Die Prüfvorrichtung ist für die Dichtigkeitsprüfung dieses medizinischen Behälters vorgesehen und ausgestaltet, den Behälter mit Fluid zu beaufschlagen und eine von der Beaufschlagung und von der Dichtigkeit des Behälters abhängige Behälterantwort zu erfassen. Die Prüfvorrichtung und der mit ihr fluidisch verbundene Behälter bilden das Prüfsystem aus. Offenbarungsgemäß ist das Fluid ein Gas.

Die erfasste Behälterantwort ist ein erfasster Druck über die Zeit, und die Prüfvorrichtung hat eine Druckerfassungseinheit, die in Verbindung mit dem Behälter ist oder zumindest bringbar ist. Im Vergleich zu der oben beschriebenen, austretenden Flüssigkeit als Behälterantwort, die visuell von einem Prüfer erfasst werden muss, ist die Erfassung des Drucks als Behälterantwort maschinell möglich, präzise und unabhängig von der Erfahrung von Bedienpersonal, so dass mit geringem Aufwand eine verlässliche Dichtigkeitsprüfung bereitgestellt ist.

Weiterhin hat die Prüfvorrichtung eine Steuereinheit, über die die Dichtigkeitsprüfung automatisiert erfolgt. In ihr sind wenigstens eine, insbesondere behälterspezifische, zeitabhängige Druckkennlinie des Behälters und eine Toleranz zu dieser Druckkennlinie hinterlegt. Die Steuereinheit ist ausgestaltet, dass über sie der erfasste Druck über die Zeit gegen die Druckkennlinie und deren Toleranz abgeglichen wird und in Abhängigkeit dieses Abgleichs, insbesondere bei Einhalten bzw. Verletzen der Toleranz, ein Prüfergebnis "dicht" oder "undicht" oder eine Fehlermeldung oder eine Information ausgegeben wird.

Vorzugsweise hat die Prüfvorrichtung eine mit der Steuereinheit signalverbundene Bedienschnittstelle, über die diese Ausgabe(n) erfolgen kann(können) und über die auch Eingaben des Bedienpersonals erfolgen können.

Besonders bevorzugt ist die Prüfvorrichtung vorgesehen und ausgestaltet, unterschiedliche oder spezifische Ausprägungen des Behälters zu prüfen.

Gemäß einer Weiterbildung der Prüfvorrichtung sind in der Steuereinheit die Druckkennlinie und deren Toleranz jeweils für unterschiedliche oder spezifische Ausprägungen des Behälters hinterlegt.

Die Druckkennlinie und Toleranz hängen von Parametern des Behälters und insbesondere einer Prüfumgebung ab. Insbesondere gehen als Parameter ein: die Form, das Material, die Materialstärke, das Volumen des Behälters, ob der Behälter befüllt/ nicht befüllt ist, die Art der Befüllung, der Grad der Befüllung, der Startdruck, ab dem der Druck über die Zeit erfasst wird, sowie eine Temperatur beim Prüfen. Dementsprechend ist die Druckkennlinie und Toleranz bezüglich dieser Parameter spezifisch und muss bei abweichenden Parametern neu ermittelt werden.

Gemäß einer Weiterbildung ist die Prüfvorrichtung ausgestaltet, insbesondere mit pneumatischen Mitteln ausgestaltet, den Behälter mit Gas zu beaufschlagen. Hierbei sind wenigstens eine Druckluftkupplung zur Verbindung mit einer Druckluftquelle und wenigstens eine Behälterkupplung zur Verbindung mit dem Behälter vorgesehen. Optional kann eine Abluftkupplung zur Verbindung mit einer Abluftsenke vorgesehen sein.

Bevorzugt ist die Druckerfassungseinheit an der Behälterkupplung angeordnet oder an dieser angesetzt, insbesondere eingeschraubt oder eingekuppelt. Der Vorteil hierbei ist, dass die Erfassung des Drucks nahe an dem Behälter erfolgt und nicht an einer entfernten Stelle oder Leitung der Prüfvorrichtung. Somit erfährt die Erfassung des Drucks eine geringstmögliche Dämpfung oder Verzögerung und eine Störanfälligkeit ist gering. Die Folge ist eine besonders präzise Erfassung. Zudem erfolgt so die Verbindung der Druckerfassungseinheit in einem gemeinsamen Schritt mit der Verbindung der Behälterkupplung und es sind keine getrennten Kupplungen notwendig, was die Fehleranfälligkeit beim Verbinden minimiert.

Die Prüfvorrichtung erweist sich als flexibel und zudem sicher in der Prüfung unterschiedlicher Behälter, wenn sie gemäß einer bevorzugten Weiterbildung mehrere unterschiedlich oder spezifisch ausgeprägte Behälterkupplungen für mehrere, unterschiedlich oder spezifisch ausgeprägte Behälter hat. Unterschiedlich oder spezifisch ist hierbei so zu verstehen, dass die jeweilig spezifisch ausgeprägte Behälterkupplung inkompatibel mit der jeweils anderen spezifischen Ausprägung des Behälters ist. In anderen Worten ist die spezifisch ausgeprägte Behälterkupplung nur mit dem ihr zugeordneten, spezifisch ausgeprägten Behälter kuppelbar ausgestaltet und mit keinem anderen.

Die Prüfvorrichtung ist auch sicherer in der Prüfung von, insbesondere unterschiedlichen, Behältern, wenn gemäß einer Weiterbildung die Behälterkupplung einen mit der Steuereinheit signalverbundenen Sensor hat, der ausgestaltet ist, zu erfassen, ob die Behälterkupplung korrekt mit dem Behälter verbunden ist.

Für diesen Fall weist gemäß einer Weiterbildung die jeweilige spezifisch ausgeprägte Behälterkupplung vorzugsweise einen spezifischen RFID-Lesekopf als Sensor auf. Vorzugsweise ist dieser dazu vorgesehen und ausgestaltet, einen spezifischen RFID-Tag des spezifisch ausgeprägten Behälters zu erfassen, auszulesen und der Steuereinheit zum einen die Information zu übermitteln, welcher spezifische Behälter verbunden ist, und zum anderen, ob die spezifische Behälterkupplung korrekt mit dem Behälter verbunden ist oder nicht.

Die Prüfvorrichtung ist auch sicherer in der Prüfung von, insbesondere unterschiedlichen, Behältern, wenn sie gemäß einer Weiterbildung einen mit der Steuereinheit signalverbundenen Sensor hat, der ausgestaltet ist, zu erfassen, ob die Behälterkupplung von einem Steckplatz der Prüfvorrichtung, an dem sie vorzugsweise bei Nichtgebrauch vorgehalten ist, entnommen ist oder nicht. Vorzugsweise ist dieser Sensor vorrichtungstechnisch einfach als Lichtschranke ausgestaltet.

Gemäß einer Weiterbildung hat die Prüfvorrichtung einen Druckluftpfad, über den die Druckluftkupplung mit der wenigstens einen Behälterkupplung verbindbar, insbesondere verbunden, ist und einen Abluftpfad, über den die wenigstens eine Behälterkupplung mit der Abluftsenke, insbesondere mit Atmosphäre verbindbar, insbesondere verbunden, ist.

Um eine Kontamination des Behälters, oder sogar der darin befindlichen medizinischen Trockensubstanz oder des medizinischen Trockenkonzentrats, zu verhindern, hat die Prüfvorrichtung gemäß einer Weiterbildung in dem Druckluftpfad eine erste Filtereinheit, insbesondere einen Sterilfilter.

Um hingegen einen Austrag der medizinischen Trockensubstanz oder des medizinischen Trockenkonzentrats zu der Abluftsenke, insbesondere in die Atmosphäre, zu verhindern, hat die Prüfvorrichtung gemäß einer Weiterbildung eine zweite Filtereinheit in dem Abluftpfad.

Eine Möglichkeit zur effizienten Schaltung des Druckluftpfades und des Abluftpfades ergibt sich, wenn die Prüfvorrichtung gemäß einer Weiterbildung eine Verzweigungsstelle oder einen Abzweig hat, an der oder dem der Abluftpfad von dem Druckluftpfad abgezweigt ist.

In einer bevorzugten Weiterbildung ist in dem Druckluftpfad zwischen dem Abzweig und der Druckluftkupplung ein Rückschlagventil angeordnet, das bei Beaufschlagung mit von dem Behälter abströmender Luft in Richtung hin zu der Druckluftkupplung schließt und bei Beaufschlagung mit Druckluft in Gegenrichtung öffnet.

Um den Druckluftpfad und den Abluftpfad individuell und sicher aufsteuern und zusteuern zu können, ist gemäß einer Weiterbildung in dem Druckluftpfad und in dem Abluftpfad jeweils ein unabhängig voneinander betätigbares Sperrventil angeordnet. Letztgenanntes ist insbesondere als elektromagnetisch betätigbares, insbesondere als 2/2-Wege-Schaltventil, ausgestaltet. Vorzugsweise ist das jeweilige Sperrventil über die Steuereinheit betätigbar.

Gemäß einer Weiterbildung hat die Prüfvorrichtung ein, insbesondere von der Steuereinheit betätigbares, Auswahlventil mit einem Versorgungsanschluss, der mit dem Druckluftpfad und dem Abluftpfad, insbesondere mit dem Abzweig, verbunden ist, und mit je einem Behälteranschluss, der mit je einer der Behälterkupplungen verbunden ist. Das Auswahlventil hat je eine betätigbare Stellung pro Behälteranschluss. Dabei ist der Versorgungsanschluss in der jeweiligen Stellung mit einem der Behälteranschlüsse verbunden, gegen die anderen Behälteranschlüsse jedoch gesperrt. Das Auswahlventil hat demzufolge bei n Behälteranschlüssen n betätigbare Stellungen.

Alternativ kann das Auswahlventil natürlich in aufgelöster Bauweise von einer Menge einzelner und einzeln betätigbarer Ventile, insbesondere von einer Menge 2/2-Wege-Schaltventilen, gebildet sein, wobei jeder Behälterkupplung dann eins der 2/2-Wege-Schaltventile zugeordnet ist.

Im Druckluftpfad ist vorzugsweise ein manuell oder elektromagnetisch einstellbares Druckregelventil angeordnet, so dass eine Druckbeaufschlagung des Behälters oder der unterschiedlichen oder spezifisch ausgeprägten Behälter mit einem präzise geregelten, insbesondere spezifischen, Druck der Druckluft erfolgen kann. Vorzugsweise ist das Druckregelventil stromaufwärts des betätigbaren Sperrventils, d.h. insbesondere zwischen der Druckluftkupplung und dem betätigbaren Sperrventil des Druckluftpfades, angeordnet.

Um den Druckluftpfad und den Abluftpfad, und insbesondere über die Stellungen des Auswahlventils den entsprechend verbundenen Behälter, gegen eine Belastung mit Überdruck abzusichern, weist die Prüfvorrichtung gemäß Weiterbildung ein manuell oder elektromagnetisch einstellbares Druckbegrenzungsventil auf, das in dauerhafter Druckluftverbindung mit dem Versorgungsanschluss ist, und das vorzugsweise im Abluftpfad angeordnet ist.

Um die Prüfung gegen Einwirkung von außen zu schützen und aber auch, um die Umgebung gegen von der Prüfung ausgehende Gefahren zu schützen, hat die Prüfvorrichtung gemäß einer Weiterbildung eine, insbesondere mobile, Einhausung mit einer Tür, durch die die Prüfvorrichtung mit dem oder den zu prüfenden Behälter oder Behältern bestückbar ist.

Vorzugsweise ist die Tür von einem Sensor, vorzugsweise mittels einem Lichtschrankensensor, auf ihre Schließstellung sensorüberwacht, wobei der Sensor mit der Steuereinheit signalverbunden ist und die Steuereinheit ausgestaltet ist, bei einer als offen sensierten Tür, die Prüfung abzubrechen, zu unterbrechen und/oder eine Warnmeldung auszugeben.

Die Tür erstreckt sich vorzugsweise anteilig, vorzugsweise an einem oberen Anteil der Einhausung, so dass an einer Unterseite ein Einblick in die Einhausung und damit auf einen Stellplatz des Behälters in der Prüfvorrichtung sichtbar ist, wodurch der Behälter insbesondere visuell identifizierbar ist. Bevorzugt deckt die Tür alle Komponenten der Prüfvorrichtung ab, die ein Risiko für Bruch aufweisen, also insbesondere Pneumatikschläuche.

Bevorzugt ist die Einhausung modular aufgebaut, wobei Kanten der Einhausung von einem Profilrahmen und Flächen der Einhausung von Platten gebildet sind, wodurch sich ein kostengünstiger und flexibler Aufbau der Einhausung ergibt. Bevorzugt ist die Einhausung quaderförmig ausgestaltet.

Um die Einhausung, beziehungsweise die Prüfvorrichtung, auf einfache Weise verlagern zu können, weisen gemäß einer Weiterbildung Basisabschnitte der Einhausung, insbesondere Endabschnitte des Profilrahmens, Rollen/ Transportrollen auf. Alternativ ist die Einhausung und damit die Prüfvorrichtung ortsfest ausgebildet.

Ein Prüfsystem zur Dichtigkeitsprüfung eines medizinischen Behälters hat gemäß der vorliegenden Offenbarung eine Prüfvorrichtung, die gemäß wenigstens einem Aspekt der vorangegangenen Beschreibung ausgestaltet ist, und einen von der Prüfvorrichtung auf Dichtigkeit zu prüfenden, medizinischen Behälter, welcher vorgesehen und ausgestaltet ist, dass in ihm eine medizinische Trockensubstanz oder ein medizinisches Trockenkonzentrat in Lösung gebracht wird.

Gemäß einer bevorzugten Weiterbildung des Prüfsystems ist eine Kupplung des Behälters mit der Behälterkupplung der Prüfvorrichtung verbunden. Vorzugsweise ist eine Kupplung der Druckluftquelle mit der Druckluftkupplung der Prüfvorrichtung und die Abluftkupplung der Prüfvorrichtung ist entweder mit einer Abluftsenke verbunden, beispielsweise mit einem Niederdruckbehälter, oder sie ist offen gegen Atmosphäre.

Gemäß einer bevorzugten Weiterbildung des Prüfsystems hat die Prüfvorrichtung mehrere unterschiedlich oder spezifisch ausgeprägte Behälterkupplungen und mehrere, unterschiedlich oder spezifisch ausgeprägte Behälter, die geprüft werden können. Dabei ist die jeweilige spezifisch ausgeprägte Behälterkupplung inkompatibel mit der jeweils anderen spezifischen Ausprägung des Behälters. In anderen Worten ist die spezifisch ausgeprägte Behälterkupplung nur mit dem ihr zugeordneten, spezifisch ausgeprägten Behälter kuppelbar ausgestaltet und mit keinem anderen.

Gemäß einer bevorzugten Weiterbildung des Prüfsystems haben die unterschiedlichen oder spezifischen Behälterkupplungen jeweils einen mit der Steuereinheit signalverbundenen Sensor, der ausgestaltet ist, zu erfassen, ob die spezifische Behälterkupplung korrekt mit dem ihr zugeordneten, spezifischen Behälter verbunden ist.

Vorzugsweise weisen die unterschiedlichen oder spezifischen Behälterkupplungen hierfür jeweils einen spezifischen RFID-Lesekopf als Sensor auf, der jeweils dazu vorgesehen und ausgestaltet ist, einen jeweils spezifischen RFID-Tag der Kupplung des spezifisch ausgeprägten Behälters zu erfassen, auszulesen und der Steuereinheit die Information zu übermitteln, welcher spezifische Behälter verbunden ist und ob die spezifische Behälterkupplung korrekt mit der Kupplung des spezifischen Behälters verbunden ist.

Ein Verfahren zur Dichtigkeitsprüfung eines medizinischen Behälters ist mit einer mit dem Behälter fluidisch verbundenen Prüfvorrichtung, in anderen Worten mit dem Prüfsystem aus Prüfvorrichtung und Behälter, vorgesehen. Bevorzugt ist die Prüfvorrichtung gemäß einem der vorhergehenden Aspekte der Beschreibung ausgestaltet. Der Behälter ist insbesondere vorgesehen und ausgestaltet, dass in ihm, wie vorbeschrieben, eine medizinische Trockensubstanz oder ein medizinisches Trockenkonzentrat, insbesondere einer Dialyse-Flüssigkeit bzw. Dialysierflüssigkeit, in Lösung gebracht werden kann. Das Verfahren weist dabei die Schritte auf:
- Beaufschlagen des Behälters mit einem Fluid mittels der Prüfvorrichtung und
- Erfassen einer Behälterantwort in Abhängigkeit der Beaufschlagung und einer Dichtigkeit des Behälters mittels einer Erfassungseinheit.

Offenbarungsgemäß erfolgt das Beaufschlagen des Behälters mit einem Gas (als Fluid), insbesondere mit Druckluft. So ist ein Verfahren für eine Trockenprüfung des Behälters auf Dichtigkeit geschaffen, wobei sich die gleichen Vorteile bezüglich des Verfahrens ergeben, wie sie bereits im Zuge der Beschreibung der Prüfvorrichtung geschildert wurden.

Die Erfassung der Behälterantwort erfolgt druckbezogen. Demgemäß ist die Erfassungseinheit eine Druckerfassungseinheit und die erfasste Behälterantwort ist ein über die Zeit erfasster Druck des Behälters, insbesondere ein erfasster Druckabfall des Behälters über die Zeit. Offenbarungsgemäß erfolgt die Prüfung auf Dichtigkeit durch Schritte:
- Abgleichen des erfassten Drucks über die Zeit gegen eine in einer Steuereinheit der Prüfvorrichtung hinterlegte, zeitabhängige Druckkennlinie des Behälters und deren Toleranz, mittels der Steuereinheit; und in Abhängigkeit des Abgleichs
- Ausgeben eines Prüfergebnisses, das den Behälter als dicht klassifiziert, bei Einhaltung der Toleranz, oder das den Behälter als undicht klassifiziert, bei Verletzung der Toleranz, oder einer Fehlermeldung oder einer Information, jeweils mittels der Steuereinheit, an eine mit ihr signalverbundene Bedienschnittstelle der Prüfvorrichtung.

Um die Prüfung durchzuführen, erfolgt vorbereitend die Kupplung des Behälters mit der Behälterkupplung der Prüfvorrichtung, sowie die Auswahl des zu prüfenden Behälters an der Bedienschnittstelle der Prüfvorrichtung. Des Weiteren wird geprüft, ob der Behälter wirklich korrekt verbunden ist und ob der ausgewählte Behälter und der verbundene Behälter übereinstimmen. Demgemäß kann das Verfahren die Schritte umfassen:
- Verbinden einer, insbesondere spezifisch ausgeprägten Behälterkupplung der Prüfvorrichtung mit einer Kupplung des, insbesondere spezifisch ausgeprägten Behälters;
- Auswählen einer Ausprägung des Behälters durch einen Bediener an der Bedienschnittstelle oder automatisiert mittels einer sensorüberwachten, mit einer Steuereinheit der Prüfvorrichtung signalverbundenen Behälterkupplung; und
- Sensorüberwachtes Prüfen, ob die Behälterkupplung korrekt mit der Kupplung verbunden ist, und/oder ob die Behälterkupplung mit der Kupplung kompatibel ist.

Falls die letztgenannte Prüfung positiv ausfällt, erfolgt ein Schritt
- Ausgeben einer Bestätigung über die Steuereinheit an die Bedienschnittstelle; und wenn nein, erfolgt ein Schritt
- Ausgeben einer Fehlermeldung über die Steuereinheit an die Bedienschnittstelle und/oder Unterbrechen der Prüfung über die Steuereinheit.

Des Weiteren kann das Verfahren folgende vorbereitende Schritte aufweisen:
- Verbinden der Druckluftkupplung der Prüfvorrichtung mit einer Kupplung einer Druckluftquelle; und
- Absperren oder Schließen aller sonstigen Anschlüsse oder Öffnungen des Behälters; sowie optional
- Verbinden einer Abluftkupplung der Prüfvorrichtung mit einer Kupplung einer Abluftsenke.

In diesem Zustand ist der Behälter korrekt angeschlossen und an der Bedienschnittstelle korrekt ausgewählt. Die Druckluftquelle und ggf. die Abluftsenke sind korrekt angeschlossen, so dass die Prüfung folglich manuell oder automatisiert gestartet werden kann. Im manuellen Fall erfolgt der Start mittels eines Bedieners an der Bedienschnittstelle und daraufhin erfolgen bevorzugt die Schritte:
- Zusteuern des Abluftpfades und somit Trennen der Behälterkupplung von der Abluftsenke, so dass der Behälter effektiv mit Druckluft beaufschlagt werden kann, ohne dass ein Bypass zur Abluftsenke entsteht. Das erfolgt bevorzugt, indem das Sperrventil des Abluftpfades von der Steuereinheit betätigt und somit zugesteuert wird;
- Aufsteuern des Druckluftpfades und somit Verbinden der Behälterkupplung mit der Druckluftquelle. Insbesondere wird hierfür das Sperrventil des Druckluftpfades mittels der Steuereinheit betätigt und aufgesteuert;
- mit Erfassen eines vorbestimmten, behälterspezifischen Start-Drucks erfolgt das Trennen der Behälterkupplung von der Druckluftquelle mittels Zusteuern des Druckluftpfades.

Bevorzugt folgen das Erfassen des Absinkens des Drucks über die Zeit und währenddessen die Schritte:
- Abgleichen des erfassten Drucks über die Zeit gegen die hinterlegte, zeitabhängige Druckkennlinie des Behälters und die Toleranz dieser Druckkennlinie; und in Abhängigkeit des Abgleichs, insbesondere in Abhängigkeit einer Einhaltung oder Verletzung der Toleranz der Schritt
- Ausgeben eines Ergebnisses "dicht" oder "undicht" und/oder einer Fehlermeldung für den Behälter.

Da der Behälter nicht starr ist, sondern in Grenzen verformbar ist und eine gewisse Formänderungsarbeit aufnehmen kann, erweist sich eine Weiterbildung des Verfahrens als vorteilhaft, in der der Behälter zunächst etwas über einen, dem Startpunkt der Druckkennlinie entsprechenden, spezifischen Start-Druck hinaus belastet wird. Demzufolge ist in der Steuereinheit ein vorbestimmter, behälterspezifischer Überdruck zum vorbestimmten, behälterspezifischen Start-Druck hinterlegt. Während die Behälterkupplung mit der Druckluftquelle verbunden ist, steigt der Druck im Behälter an und wird entsprechend erfasst. Das Trennen der Behälterkupplung von der Druckluftquelle erfolgt gemäß der Weiterbildung erst dann, wenn der vorbestimmte, behälterspezifische Überdruck erfasst wird. Daraufhin erfolgt das Absinken und das Erfassen des Drucks über die Zeit.

Bevorzugt erfolgt daraufhin das Absinken geringfügig unter den spezifischen StartDruck. Dementsprechend ist in der Steuereinheit ein vorbestimmter, behälterspezifischer Unterdruck zum vorbestimmten, behälterspezifischen Startdruck hinterlegt. Mit dem Erfassen dieses vorbestimmten, behälterspezifischen Unterdrucks erfolgt dann das erneute Verbinden der Behälterkupplung mit der Druckluftquelle, so dass in Folge der Druck wieder in Richtung des vorbestimmten, behälterspezifischen Start-Drucks ansteigt.

Mit dessen Erfassung erfolgen dann das Trennen der Behälterkupplung von der Druckluftquelle, das Erfassen des Drucks über die Zeit, der Abgleich des Drucks über die Zeit und in Folge das Ausgeben des Ergebnisses.

Gemäß einer Weiterbildung des Verfahrens kann die Aufnahme der vorbestimmten, behälterspezifischen Druckkennlinie und ihrer Toleranz mit folgenden Schritten erfolgen:
- Erfassen und Hinterlegen einer spezifischen Druckkennlinie eines dichten Behälters in der Steuereinheit;
- Einbringen eines definierten Lecks in den zuvor dichten Behälter;
- Erfassen des Drucks über die Zeit des leckagebehafteten Behälters und Hinterlegen in der Steuereinheit; und
- Nassprüfen des leckagebehafteten Behälters und Ermitteln, ob ausreichende Dichtigkeit gegeben ist.

Sollte keine ausreichende Dichtigkeit gegeben sein, erfolgen Schritte:
- Verkleinern des Lecks; und
- Wiederholen des Erfassens des Drucks über die Zeit des leckagebehafteten Behälters und Hinterlegen in der Steuereinheit; des Nassprüfens des leckagebehafteten Behälters und des Ermittelns, ob ausreichende Dichtigkeit gegeben ist.

Dies wird wiederholt, bis bei der Nassprüfung ausreichende Dichtigkeit festgestellt wird, die Nassprüfung also als erfolgreich gilt. Dann erfolgt ein Schritt:
- Ablegen des zuletzt erfassten Drucks über die Zeit als Toleranz der spezifischen Druckkennlinie des dichten Behälters in der Steuereinheit.

Wie bereits erwähnt, ist die Druckkennlinie spezifisch für die Ausprägung des Behälters. Hierunter ist zu verstehen, dass die Druckkennlinie von mehreren Parametern des Behälters abhängt. Insbesondere gehen als Parameter ein: die Form, das Material, die Materialstärke, das Volumen des Behälters, ob der Behälter befüllt/nicht befüllt ist, die Art der Befüllung, der Grad der Befüllung, der Startdruck oder der Betriebsdruck. Als Umgebungsparameter gehen ein, die Temperatur beim Prüfen, sowohl der Umgebung, des Behälters als auch des Fluides, also der Druckluft aber auch des Wassers im Falle der Nassprüfung. Dementsprechend ist die Druckkennlinie bezüglich dieser Parameter spezifisch und muss für im Prüfbetrieb zu stark abweichende Parameter ggf. neu ermittelt werden.

Für einen Behälter mit vergleichsweise dünnwandiger Bauweise liegt der vorbestimmte, behälterspezifische Startdruck zwischen 0,15 und 0,30 bar, bevorzugt zwischen 0,20 und 0,25 bar, besondere bevorzugt bei etwa 0,23 bar. Für einen Behälter mit vergleichsweise dickwandiger Bauweise liegt der vorbestimmte, behälterspezifische Startdruck zwischen 0,7 und 0,9 bar, bevorzugt zwischen 0,75 und 0,85 bar, besondere bevorzugt bei etwa 0,8 bar.

### Kurzbeschreibung der Figuren

Die Erfindung wird nachfolgend anhand bevorzugter Ausführungsformen mit Hilfe von Figuren näher erläutert. Es zeigen:
Fig. 1 zeigt in einer perspektivischen Ansicht eine Prüfvorrichtung gemäß einer ersten bevorzugten Ausführungsform der vorliegenden Offenbarung;
Fig. 2 zeigt in einer perspektivischen Teilansicht eine Prüfvorrichtung gemäß einer zweiten bevorzugten Ausführungsform der vorliegenden Offenbarung;
Fig. 3 zeigt in einer Ansicht von vorne die Prüfvorrichtung gemäß Figur 1, bestückt mit einem zu prüfenden Behälter;
Fig. 4 zeigt in einer Ansicht von vorne die Prüfvorrichtung gemäß Figur 3, mit in der Darstellung entfernter Tür;
Fig. 5 zeigt in einer Ansicht von der Seite die Prüfvorrichtung gemäß Figur 3;
Fig. 6 zeigt eine für beide Ausführungsformen der Prüfvorrichtung gemäß den Figuren 1 bis 5 gültige, pneumatische Schaltstruktur;
Fig. 7 zeigt in einer Ansicht von vorne pneumatische Mittel der Prüfvorrichtung gemäß Figur 1 zur Beaufschlagung des Behälters mit Druckluft;
Fig. 8 zeigt ein Flussdiagramm eines Verfahrens zur Dichtigkeitsprüfung gemäß einer bevorzugten Ausführungsform der vorliegenden Offenbarung;
Fig. 9 zeigt eine spezifische, zeitabhängige Druckkennlinie eines dichten Behälters erster Ausprägung, sowie Druckverläufe über die Zeit dieses Behälters mit unterschiedlich großen Lecks; und
Fig. 10 zeigt eine spezifische, zeitabhängige Druckkennlinie eines dichten Behälters zweiter Ausprägung, sowie Druckverläufe über die Zeit dieses Behälters mit unterschiedlich großen Lecks.

Die Figuren sind schematischer Natur und sollen nur dem Verständnis der Erfindung dienen. Gleiche Elemente sind mit denselben Bezugszeichen versehen. Die Merkmale der verschiedenen Ausführungsbeispiele können untereinander ausgetauscht werden.

### Detaillierte Beschreibung bevorzugter Ausführungsformen

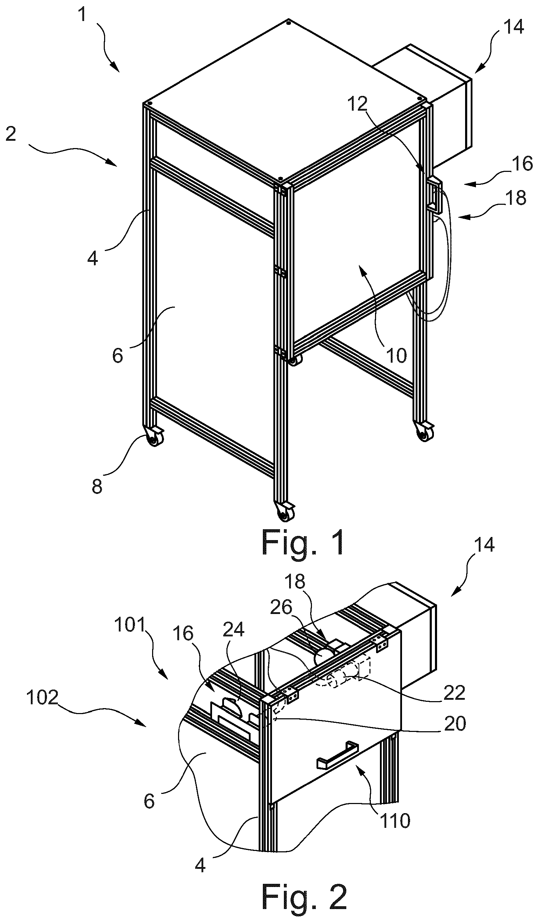

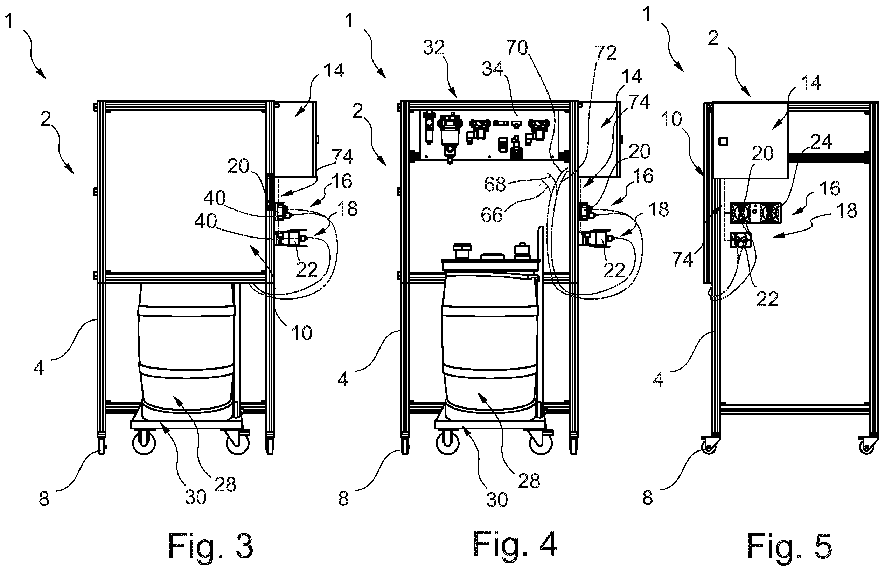

Figur 1 zeigt in einer perspektivischen Ansicht eine Prüfvorrichtung 1 zur Dichtigkeitsprüfung eines medizinischen Behälters, der zur Lösung von medizinischem Trockenkonzentrat vorgesehen bzw. ausgestaltet ist (vgl. Figuren 3 und 4). Gemäß Figur 1 ist die Prüfvorrichtung 1 ohne den Behälter dargestellt. Sie hat ein in der gezeigten Ausführungsform quaderförmiges Gehäuse, bzw. eine quaderförmige Einhausung 2, die zur Aufnahme des Behälters vorgesehen ist, und die die zur Dichtigkeitsprüfung vorgesehenen, pneumatische Komponenten aufnimmt und schützt. Die Einhausung 2 ist in Rahmenbauweise ausgeführt und ist aus Rahmenprofilen 4, die die Quaderkanten ausbilden, und Platten 6, die die Quaderflächen ausbilden, zusammengesetzt. Fußseitig, d.h. an bodenseitigen Endabschnitten der vier vertikalen Rahmenprofile 4 sind diese mit je einer Bodenrolle 8 bestückt. So ist die Einhausung 2 und damit die Prüfvorrichtung 1 mobil und auf einfache Weise an einen anderen Standort verschiebbar.

Vorderseitig hat die mobile Einhausung 2 eine in der Ausführungsform gemäß Figur 1 vertikal links anscharnierte Tür 10, die in Figur 1 geschlossen dargestellt ist, und die den Zugang in die Einhausung 2 zum Bestücken der Prüfvorrichtung 1 mit dem zu prüfenden Behälter ermöglicht. Die Tür 10 erstreckt sich gemäß Figur 1 lediglich über eine obere Hälfte einer Vorderseite der Prüfvorrichtung 1. Im unteren Teil der Vorderseite weist die Einhausung 2 somit eine Lücke auf, durch die der Blick ins Innere der Einhausung freigegeben ist. Durch diese erfolgt bei Bedarf eine einfache Sichtkontrolle, ob und ggf. mit welchem Behälter die Prüfvorrichtung 1 bestückt ist. Die Tür kann mittels einem rechts angeordneten Griff 12 betätigt werden. Die Tür 10 ist mittels einem Lichtschrankensensor sensorüberwacht und eine Information über ihren Öffnungs- oder Schließzustand wird permanent an eine elektronische Steuereinheit der Prüfvorrichtung 1 übermittelt. Die Prüfung kann dabei nur gestartet werden, wenn die Tür 10 als geschlossen detektiert und gemeldet wird.

Seitlich an der Einhausung ist ein elektrischer Steuerungskasten 14 vorgesehen, der unter anderem die elektronische Steuereinheit beinhaltet, mittels der die Prüfvorrichtung 1 und ihre pneumatischen Mittel zum Zwecke der Dichtigkeitsprüfung gesteuert werden, und die einer Bedienschnittstelle der Prüfvorrichtung 1 ein Ergebnis der Dichtigkeitsprüfung bereitstellt. Unterhalb des elektrischen Steuerungskastens 14 sind an der Außenseite der Einhausung 2 zwei Steckplätze 16, 18 unterschiedlicher oder spezifischer Ausprägung vorgesehen, an denen jeweils eine spezifisch ausgeprägte Behälterkupplung (in Fig. 1 nicht gezeigt, vgl. 20, 22 in Fig. 3 bis 5) für einen medizinischen Behälter spezifischer Ausprägung vorgehalten ist.

Figur 2 zeigt in einer perspektivischen Teilansicht einer abweichend ausgestalteten Einhausung 102 einer Prüfvorrichtung 101 gemäß einer zweiten bevorzugten Ausführungsform der vorliegenden Offenbarung, wobei abweichend von der ersten Ausführungsform die Tür 110 horizontal anscharniert ist und eine geringere vertikale Ausdehnung aufweist. Die Deckplatte und eine obere Seitenplatte der Einhausung 102 sind in der Darstellung entfernt, sodass ein Blick ins obere Innere der Einhausung 102 freigegeben ist. Im Unterschied zur Prüfvorrichtung 1 gemäß Figur 1 sind die unterschiedlichen oder spezifischen Steckplätze 16 und 18 mit der jeweils spezifisch ausgeprägten Behälterkupplung 20, bzw. 22 und einem jeweils spezifisch ausgeprägten Behälterstopfen 24, bzw. 26 innerhalb der Einhausung 102 vorgehalten.

Der Behälterstopfen 24, bzw. 26 ist zum Verschluss eines Steigrohranschlusses des Behälters bei der Dichtigkeitsprüfung und die Behälterkupplung 20, bzw. 22 ist zur Beaufschlagung des Behälters mit Druckluft und zur dabei erfolgenden Messung des Drucks des Behälters vorgesehen.

Jede Behälterkupplung 20, bzw. 22 und jeder Behälterstopfen 24, bzw. 26 ist bezüglich Geometrie und Formschluss an eine spezifische, behälterseitige Kupplung des jeweiligen Behälters spezifischer Ausprägung angepasst. Auf diese Weise sind die Behälterkupplung 20, bzw. 22 und der Behälterstopfen 24, bzw. 26 nur mit dem ihnen zugeordneten, spezifisch ausgeprägten Behälter verbindbar und ansonsten inkompatibel, sodass eine versehentlich falsche Verbindung/ Kopplung ausgeschlossen ist.

Figur 3 zeigt in einer Ansicht von vorne die Prüfvorrichtung 1 gemäß Figur 1, bestückt mit einem zu prüfenden Behälter 28, der auf einem Rollwagen 30 angeordnet ist. Gemäß Figur 3 sind die Steckplätze 16, 18 außen an der Einhausung 2, unterhalb des Steuerungskasten 14 vorgesehen, wobei die spezifische Behälterkupplung 20 erster Ausprägung den oberen Steckplatz 16 und die spezifische Behälterkupplung 22 zweiter Ausprägung den unteren Steckplatz 18 belegt.

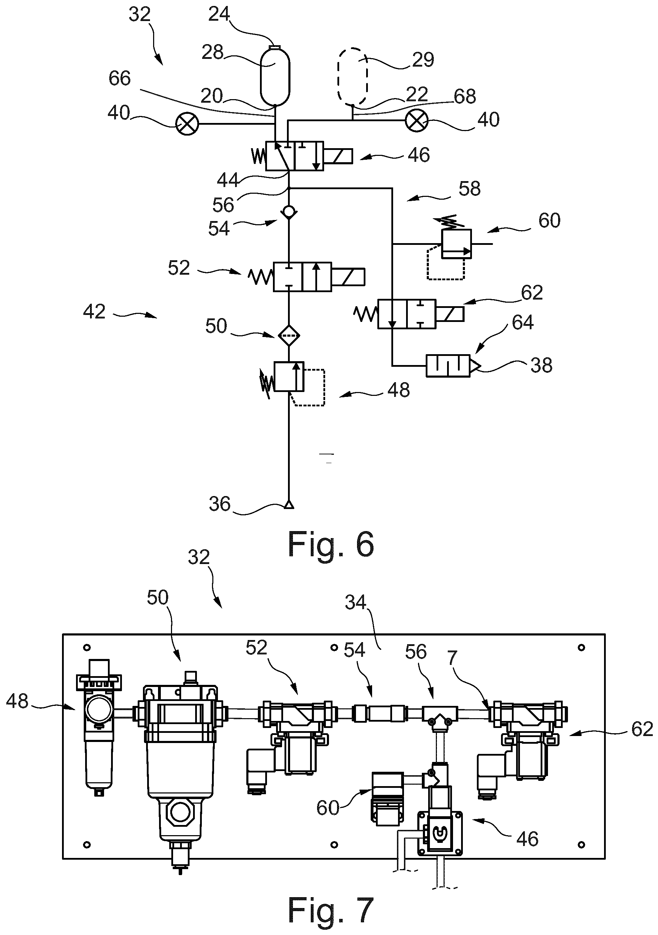

Figur 4 zeigt in einer Ansicht von vorne die Prüfvorrichtung 1 gemäß Figur 3, mit in der Darstellung entfernter Tür 10. So ist der Blick in die Einhausung 2 mit dem darin angeordneten Behälter 28 vollständig freigegeben. Am rückseitigen, oberen Wandabschnitt ist in der Einhausung 2 eine pneumatische Steueranordnung 32 auf einer Grundplatte 34 angeordnet, die anhand der Figuren 6 und 7 näher erläutert wird.

Unabhängig vom jeweiligen Ausführungsbeispiel ist die Behälterkupplung 20, bzw. 22 gemäß den Figuren 1 bis 6 mittels einem Pneumatikschlauch 66, bzw. 68 mit der pneumatische Steueranordnung 32 pneumatisch verbunden. Zudem ist ein jeweiliger Druck-Sensor 40 und ein jeweiliger RFID-Sensor (innerhalb der jeweiligen Behälterkupplung, d.h. verdeckt) der Behälterkupplung 20, bzw. 22 gemäß den Figuren 1 bis 6 mittels einem Signalleitungsbündel 70, bzw. 72 mit der im elektrischen Steuerungskasten 14 angeordneten Steuereinheit signalverbunden. Der jeweilige Drucksensor 40 dient dem Abgriff des Drucks in dem zu prüfenden Behälter 28, bzw. 29. Der RFID-Lesekopf erfasst einen RFID-Tag an der spezifischen Kupplung des spezifischen Behälters 28, bzw. 29, mit der die spezifische Behälterkupplung 20, bzw. 22 verbunden wird, sodass sowohl eine korrekte und eine fehlerhafte Verbindung der Behälterkupplung 20, bzw. 22 mit der Kupplung des Behälters 28, bzw. 29 an die Steuereinheit signalisierbar ist.

Mittels einem jeweiligen Lichtschrankensensor (verdeckt von der jeweiligen Behälterkupplung 20, bzw. 22) wird erfasst, ob der jeweilige Steckplatz 16, bzw. 18 von der zugeordneten Behälterkupplung 20, bzw. 22 belegt ist, oder nicht. Die Lichtschrankensensoren sind über Signalleitungen 74 mit der Steuereinheit im elektrischen Steuerungskasten 14 signalverbunden.

Figur 5 zeigt in einer Ansicht von der Seite die unbestückte Prüfvorrichtung 1 gemäß Figur 3 und 4 mit geschlossener Tür 10. Hierbei sind die spezifischen Steckplätze 16 und 18 in der Draufsicht erkennbar. Der Steckplatz 18 weist dabei lediglich die Behälterkupplung 22 auf. Das liegt darin begründet, dass in diesem Fall der Behälterstopfen 24 nicht spezifisch ist, sondern zum Verschluss beider spezifisch ausgeprägten Behälter verwendet wird.

Figur 6 zeigt einen Schaltplan der pneumatischen Steueranordnung 32 gemäß Figur 4 und 7. Die Beschreibung der pneumatischen Steueranordnung 32 erfolgt anhand Figur 6 schematisch, während Figur 7 gleichzeitig die physische Anordnung der beschriebenen Komponenten auf der Grundplatte 34 verdeutlicht.

Gemäß der Figur 6 mit Bezug zu Figur 7 hat die Steueranordnung 32 eine Druckluftkupplung 36 zur Verbindung mit einer Druckluftquelle, beispielsweise auf einem Druckniveau von 8 bar, eine Abluftkupplung 38 zur Verbindung mit einer Abluftsenke, sowie in der gezeigten Ausführungsform die zwei spezifischen Behälterkupplungen 20, 22. In der Darstellung gemäß Figur 6 ist die spezifische Behälterkupplung 20 mit dem spezifisch ausgeprägten Behälter 28 verbunden, dessen Steigrohranschluss vom Behälterstopfen 24 verschlossen ist. Der spezifisch ausgeprägte Behälter 29 ist hingegen nicht angeschlossen, ist aber aus Gründen der Veranschaulichung gestrichelt dargestellt. Jeder der spezifischen Behälterkupplungen 20, 22 ist eine Druckerfassungseinheit 40 zugeordnet. Von der Druckluftkupplung 36 erstreckt sich ein Druckluftpfad 42 hin zu einem Versorgungsanschluss 44 eines elektromagnetisch betätigbaren 3/2-Wege-Schaltventils 46. Ausgehend von der Druckluftkupplung 36 sind im Druckluftpfad 42 ein einstellbares Druckregelventil 48, ein Sterilfilter 50, ein elektromagnetisch betätigbares 2/2-Wege-Schaltventil 52 (Sperrventil), ein in Richtung hin zur Druckluftkupplung 36 schließendes Rückschlagventil 54 und ein Abzweig 56 eines Abluftpfades 58 angeordnet. Der Druckluftpfad endet am Versorgungsanschluss 44. Mit dem Abluftpfad 58 ist ein einstellbares Druckbegrenzungsventil 60 in dauerhafter Verbindung, über das der Druckluftpfad, die Behälter 28, 29 und der Abluftpfad gegen Überdruck abgesichert sind. Im Abluftpfad 58 ist des Weiteren ein elektromagnetisch betätigbares 2/2-Wege-Schaltventil 62 (Sperrventil) und stromab davon ein Abluftfilter 64 angeordnet, der den Austrag von Trockensubstanz in die Atmosphäre verhindert. Der Abluftpfad endet an der Abluftkupplung 38. Das Auswahlventil 46 hat zwei Behälteranschlüsse, die jeweils mit einer der spezifischen Behälterkupplungen 20, 22 verbunden sind, und zwei Schaltstellungen. In der jeweiligen Schaltstellung ist einer der Behälteranschlüsse mit dem Versorgungsanschluss 44 verbunden, während der jeweils andere Behälteranschluss gegen den Versorgungsanschluss 44 abgesperrt ist.

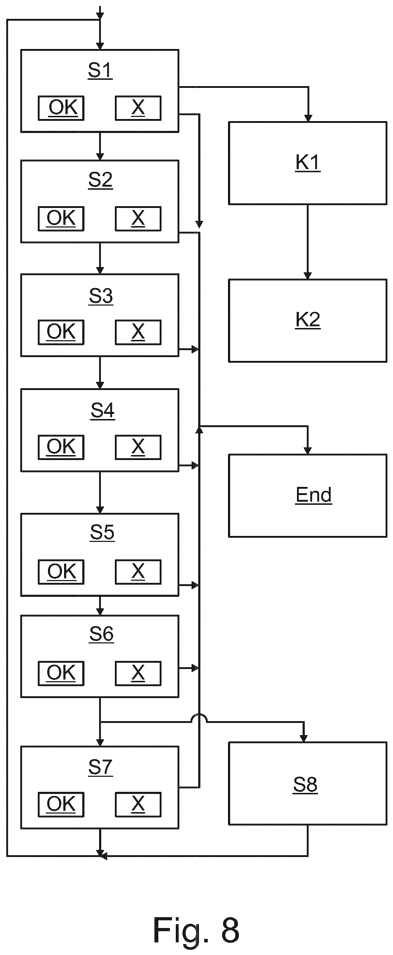

Figur 8 zeigt ein Flussdiagramm eines Verfahrens zur Dichtigkeitsprüfung gemäß einer bevorzugten Ausführungsform der vorliegenden Offenbarung.

Vorbereitend wird die Prüfvorrichtung 1 an eine Versorgungsspannung von 230VAC angeschlossen und die Druckluftkupplung 36 gemäß Figur 6 wird an eine Druckluftquelle von vorzugsweise 8 bar angeschlossen. Optional kann die Prüfvorrichtung 1 beispielsweise über Ethernet an ein lokales Netzwerk angeschlossen werden, um Datenprotokolle der Steuereinheit auszulesen. Eine Befehlseingabe durch einen Bediener erfolgt über eine Bedienschnittstelle, insbesondere eine SPS mit einem Touchscreen. Vorbereitend wird weiterhin über die sensorüberwachte Tür 10; 110 gemäß den Figuren 1, 3; 2 der Behälter in die Prüfvorrichtung 1 eingeschoben. Je nach Ausprägung des Behälters 28 bzw. 29 steht die spezifisch ausgeprägte, am Steckplatz 16, bzw. 18 vorgehaltene, sensorüberwachte Behälterkupplung 20, bzw. 22 zur Verfügung. Der spezifische Behälter 28, bzw. 29 wird mit der spezifischen Behälterkupplung 20, bzw. 22 verbunden und die Tür 10, bzw. 110 wird geschlossen.

Gemäß einer Ausführungsform des Verfahrens prüft die Prüfvorrichtung 1, ob der verbundene Behälter 28, bzw. 29 mit dem ausgewählten Behälter übereinstimmt und ob die Tür 10, bzw. 110 sicher verschlossen ist. Über das Druckregelventil 48 wird der Eingangsdruck auf einen definierten Maximaldruck eingestellt. Das 2/2-Wegeventil 52 öffnet zu Beginn der Prüfung den Druckluftpfad 42, das 3/2-Wegeventil 46 verbindet die spezifische Behälterkupplung 20, bzw. 22 mit dem Druckluftpfad 42. Das 2/2-Wegeventil 62 sperrt gleichzeitig den Abluftpfad, so dass die Luft in den Behälter 28, bzw. 29 geleitet wird. Über die Druckerfassungseinheit 40, die in der Behälterkupplung 20, bzw. 22 des Behälters 28, bzw. 29 angeordnet ist, wird der Druck erfasst und von der Steuereinheit (SPS) mit der spezifischen Druckkennlinie und deren Toleranz abgeglichen. Am Ende erfolgt in Abhängigkeit des Abgleichs die Ausgabe einer Bewertung des geprüften Behälters 28, bzw. 29. Über die stromlos geschalteten Sperrventile 52 und 62 wird der Druckluftpfad 42 abgesperrt und der Abluftpfad geöffnet, sodass der Behälter 28, bzw. 29 drucklos gemacht wird. Der Behälter 28, bzw. 29 wird abgeklemmt und der Prüfvorrichtung 1 entnommen und die Prüfvorrichtung 1 wieder freigegeben.

Es folgt ein Schritt S1 Starten der Prüfung und Auswählen des spezifischen Behälters 28, bzw. 29. Außerdem gelangt ein Bediener über die Bedienschnittstelle in Konfigurationsmenüs K1 und K2 der Bedienschnittstelle. Hier können Konfigurationen bezüglich des ausgewählten Behälters 28, bzw. 29, der Prüfvorrichtung 1 und des Prüfverfahrens eingesehen, ausgewählt oder gewechselt werden. Diese sind das Betätigung des Sperrventils 52 im Druckluftpfad 42 für das Befüllen (stromlos geschlossen), das Betätigung des Sperrventils 62 im Abluftpfad 58 für das Entleeren (stromlos geöffnet), das Betätigung zum Umschalten des Auswahlventils 46, eine Fülldauer in Sekunden, eine Prüfdauer in Sekunden, der erfasste Druck in Echtzeit, ein Solldruck, die Toleranz in %, der Überdruck, Statusanzeigen zu dejeweils sensorüberwachten Behälterkupplung, etc.

An der Bedienschnittstelle erfolgt die Fortführung des Verfahrens durch Drücken des Buttons OK.

Nach diesen Prüfungsvorbereitungen kann nun die Dichtigkeitsprüfung gestartet werden. Das kann durch die Entnahme der spezifischen Behälterkupplung 20, bzw. 22 vom mittels einer Lichtschranke sensorüberwachten Steckplatz 16, bzw. 18 erfolgen. Durch das Signal der betreffenden Lichtschranke wird automatisch der spezifische Behälter zur Prüfung ausgewählt. Sollte dies nicht der Fall sein, kann er manuell über die Bedienschnittstelle ausgewählt werden.

Es folgt eine Abfrage S3, ob der zu prüfende Behälter 28, bzw. 29 korrekt angeschlossen ist. Wird die Abfrage mit OK bestätigt, wird der Behälter 28, bzw. 29 mit der Druckluftquelle verbunden und befüllt, bis zu einem Überdruck oberhalb eines spezifischen, insbesondere eines der spezifischen Ausprägung des Behälters entsprechenden, Start-Drucks. Hierbei liegen Werte des Überdrucks beispielsweise bei 0,05 bar oberhalb des Start-Drucks, beispielsweise bei 0,25 zu 0,2 bar für den Behälter erster Ausprägung 28 und bei 0,85 zu 0,8 bar für den Behälter zweiter Ausprägung 29, damit sich der jeweilige Kunststoffbehälter, an seine Bauweise angepasst, ausdehnen kann.

Anschließend wird im Schritt S4 gewartet, bis der erfasste Druck auf einen Wert geringfügig unterhalb des Start-Drucks abgesunken ist (Unterdruck, unterhalb des Start-Drucks). Dieser sogenannte Unterdruck liegt beispielsweise 0,002 bar unterhalb des Start-Drucks, beispielsweise bei 0,198 zu 0,2 bar für den Behälter erster Ausprägung 28 und bei 0,798 zu 0,8 bar für den Behälter zweiter Ausprägung 29.

Mit Erfassen dieses Unterdrucks, erfolgt in einem Schritt S5 wieder die Verbindung des Behälters 28, bzw. 29 mit der Druckluftquelle, bis der für die Prüfung spezifische StartDruck (0,2, bzw. 0,8) erfasst wird. Mit dessen Erfassung wird der Behälter 28, bzw. 29 von der Druckluftquelle getrennt und es erfolgt die Erfassung des Drucks über die Zeit.

Im Schritt S6 erfolgt der Abgleich des erfassten Drucks über die Zeit des Behälters 28, bzw. 29 gegen die hinterlegte, spezifische Druckkennlinie und die Toleranz dieser Druckkennlinie. Die Prüfzeit beträgt dabei beispielsweise 60 Sekunden.

Ermittelt die Steuereinheit die Einhaltung der Toleranz innerhalb der Prüfzeit, erfolgt die Ausgabe des positiven Ergebnisses an die Bedienschnittstelle. Im anderen Fall erfolgt eine Abfrage S7, ob die Prüfung erneut durchgeführt werden soll oder ob sie abgebrochen werden soll. Im Falle des ausgewählten Abbruchs wird das Sperrventil 62 im Abluftpfad 58 stromlos geschaltet und somit der Behälter 28, bzw. 29 mit der Abluftsenke verbunden, so dass die Druckluft aus dem Behälter 28, bzw. 29 abströmen kann.

Gemäß dem Schritt S8 wird bei nicht bestandener Prüfung abgefragt, ob die Prüfung erneut erfolgen soll oder ob komplett abgebrochen werden soll.

Jederzeit kann das Verfahren mit Druck auf einen Abbruchbutton X der Bedienschnittstelle abgebrochen werden, sodass die Dichtigkeitsprüfung abgebrochen wird (End).

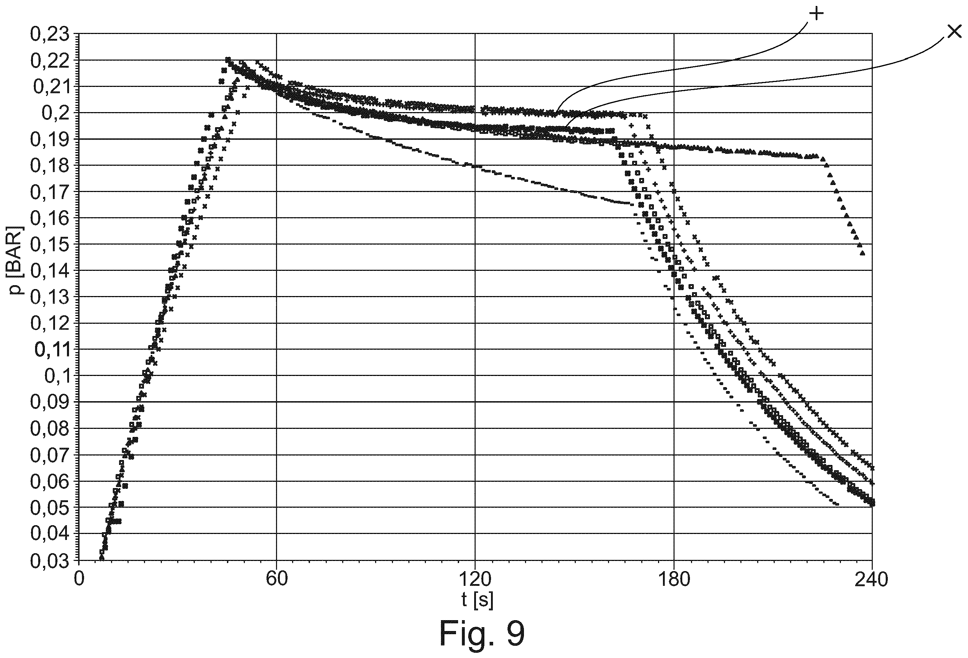

Figur 9 zeigt eine spezifische, zeitabhängige Druckkennlinie "+" des dichten Behälters erster Ausprägung 28, sowie deren Toleranz "x" oder Toleranzkurve, die ein zu prüfender Behälter 28 während der Prüfung auf Dichtigkeit nicht unterschreiten darf, um als ausreichend dicht bewertet zu werden. Die Ermittlung der Druckkennlinie "+" und ihrer Toleranz "x" basiert dabei auf folgender Vorgehensweise. Zunächst erfolgt die Beaufschlagung des dichten Behälters 28 erster Ausprägung mit Druckluft bis auf den Start-Druck von 0,2 bar. Bei abgesperrtem Druckluftpfad 42 und Abluftpfad 58 erfolgt die Erfassung des Drucks über die Zeit und die Aufnahme der spezifischen Druckkennlinie "+". Diese zeigt einen sehr geringen Druckabfall über die Zeit, was die hohe Dichtigkeit des Behälters repräsentiert. Danach wurde ein Loch mit einem Durchmesser von 1mm in eine oberseitige Schweißnaht des Behälters 28 eingebracht und die Dichtigkeitsprüfung mit Erfassung des Drucks über die Zeit wurde wiederholt. Nun ergibt sich gemäß Figur 9 unten die Kurve mit dem steilsten Druckabfall. Um dieses erzeugte Leck weiter zu verkleinern, wurden nun sequentiell Metallstifte mit ansteigendem Durchmesser in die Bohrung gesteckt und die Prüfung mit Erfassung des Drucks über die Zeit wurde jeweils durchgeführt. Mit größeren Durchmesser des eingebrachten Metallstiftes erfolgte demnach eine zunehmende Verkleinerung des Lecks, sodass die folgend aufgenommenen Kurven einen immer geringeren Druckabfall zeigen. Als nächstes wurde ein Metallstift mit einem Durchmesser von 0,9mm in die Bohrung gesteckt und es ergibt sich ein Druck über die Zeit, der keinen Unterschied zum dichten Fass erkennen lässt. Der Behälter wurde daraufhin einer Nassprüfung mit in ihm gelösten Trockenkonzentrat unterzogen und das Leck zeigte keinen Austritt. Um das Leck wieder zu vergrößern, wurde ein 0,8mm-Metallstift in die Bohrung gesteckt. Der dann bei der folgenden Prüfung erfasste Druck über die Zeit "x" zeigt gerade noch eine Abweichung zur spezifischen Druckkennlinie "+" des dichten Behälters 28. Diese Leckgröße stellt somit die Grenze eines gerade noch detektierbaren Lecks dar und der zugeordnete, erfasste Druck über die Zeit "x" stellt die Toleranz zur spezifischen Druckkennlinie "+" dar, die ein Prüfling des spezifischen Behälters 28 nicht unterschreiten darf, um als "dicht" klassifiziert zu werden.

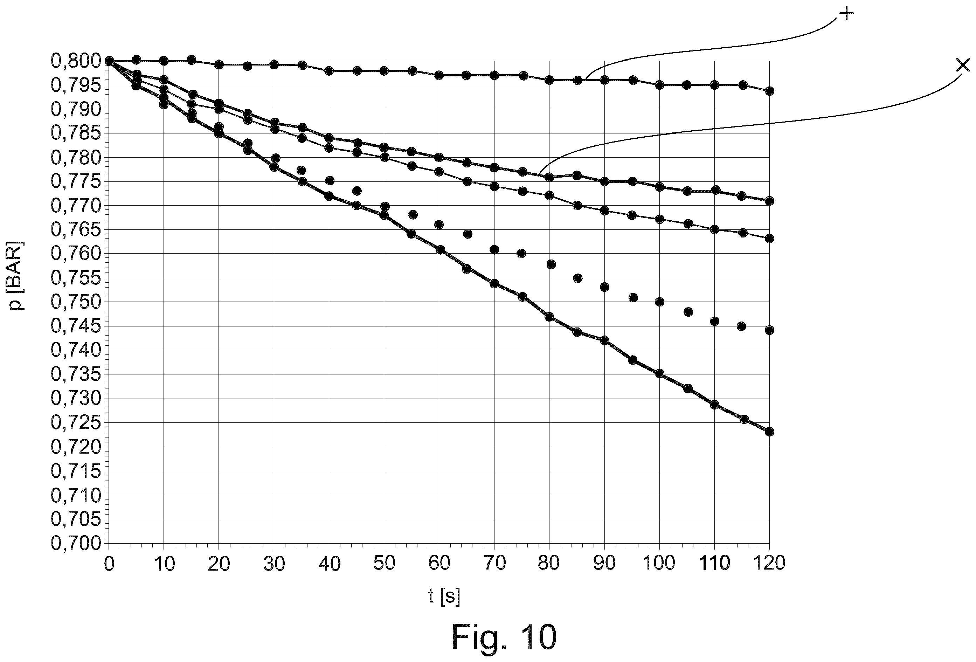

Figur 10 zeigt eine spezifische, zeitabhängige Druckkennlinie "+" eines dichten Behälters zweiter Ausprägung 29, sowie deren Toleranz "x" oder Toleranzkurve, die ein Prüfling des spezifischen Behälters 29 während der Prüfung auf Dichtigkeit nicht unterschreiten darf, um als ausreichend dicht bewertet zu werden. Die Ermittlung der spezifischen Druckkennlinie "+", sowie ihrer Toleranz "x", erfolgte analog zu dem Vorgehen, das mit Bezug zu dem Behälter erster Ausprägung 28 anhand der Figur 9 beschrieben wurde. Aufgrund der größeren Wandstärke des Behälters zweiter Ausprägung 29 erfolgt die Dichtigkeitsprüfung mit einem erhöhten, spezifischen StartDruck von 0,8 bar. Zwar zeigt die Toleranz "x" gemäß Figur 10 verglichen mit derjenigen gemäß Figur 9 einen bedeutend markanteren Druckabfall, allerdings konnte bei der folgenden Nassprüfung kein Austritt erkannt werden, sodass der Druck über die Zeit gemäß der Kurve "x" als Toleranz der Druckkennlinie "+" des Behälters zweiter Ausprägung 29 definiert ist.

### Bezugszeichenliste

- 1; 101: Prüfvorrichtung
- 2; 102: Einhausung
- 4: Profil
- 6: Platte
- 8: Rolle
- 10; 110: Tür
- 12: Griff
- 14: Steuerungskasten
- 16, 18: Kupplungssteckplatz
- 20: Behälterkupplung erster Ausprägung
- 22: Behälterkupplung zweiter Ausprägung
- 24: Behälterstopfen erster Ausprägung
- 26: Behälterstopfen zweiter Ausprägung
- 28: Behälter erster Ausprägung
- 29: Behälter zweiter Ausprägung
- 30: Rollwagen
- 32: pneumatische Steueranordnung
- 34: Grundplatte
- 36: Druckluftkupplung
- 38: Abluftkupplung
- 40: Druckerfassungseinheit
- 42: Druckluftpfad
- 44: Versorgungsanschluss
- 46: 3/2-Wege-Auswahlventil
- 48: Druckregelventil
- 50: Sterilfilter
- 52: Sperrventil
- 54: Rückschlagventil
- 56: Abzweig
- 58: Abluftpfad
- 60: Druckbegrenzungsventil
- 62: Sperrventil
- 64: Abluftfilter
- 66, 68: Pneumatikschlauch
- 70, 72: Signalleitungsbündel Druck-Sensor/ RFID-Sensor

- S1: Starten der Dichtigkeitsprüfung eines Behälters
- S2: Auswählen eines spezifischen Behälters
- S3: Druck oberhalb Start-Druck
- S4: Warten bis Druck unterhalb Startdruck
- S5: Druck auf Start-Druck und Erfassen des Drucks über die Zeit
- S6: Abgleich Druck über die Zeit gegen Druckkennlinie und Toleranz
- S7: Ausgabe Ergebnis
- S7: Abfrage erneutes Prüfen

- K1, K2: Konfiguration Behälter, Prüfvorrichtung, Prüfverfahren

- +: Druckkennlinie
- x: Toleranz(kurve)

## Patentansprüche

1. Prüfvorrichtung zur Dichtigkeitsprüfung eines medizinischen Behälters (28, 29), welcher vorgesehen und ausgestaltet ist, in ihm eine medizinische Trockensubstanz oder ein medizinisches Trockenkonzentrat in Lösung zu bringen, wobei die Prüfvorrichtung (1; 101) vorgesehen und ausgestaltet ist, den Behälter (28, 29) mit einem Gas zu beaufschlagen und eine von der Beaufschlagung und einer Dichtigkeit des Behälters (28, 29) abhängige Behälterantwort zu erfassen, wobei die Behälterantwort ein Druck über die Zeit ist, und wobei die Prüfvorrichtung (1; 101) zur Erfassung der Behälterantwort eine Druckerfassungseinheit (40) hat, die zur Verbindung mit dem Behälter (28, 29) vorgesehen und ausgestaltet ist, **gekennzeichnet durch** eine Steuereinheit, in der eine zeitabhängige Druckkennlinie (+) des Behälters (28, 29) und eine Toleranz (x) zu dieser Druckkennlinie (+) hinterlegt sind, und die ausgestaltet ist, den erfassten Druck über die Zeit gegen die zeitabhängige Druckkennlinie (+) auf eine Einhaltung der Toleranz (x) abzugleichen und in Abhängigkeit des Abgleichs ein Prüfergebnis "dicht" oder "undicht" oder eine Fehlermeldung oder eine Information auszugeben.

2. Prüfvorrichtung nach Anspruch 1, **dadurch gekennzeichnet, dass** in der Steuereinheit die Druckkennlinie (+) und Toleranz (x) jeweils für spezifische Ausprägungen des Behälters (28, 29) hinterlegt sind.

3. Prüfvorrichtung nach einem der vorhergehenden Ansprüche, **dadurch gekennzeichnet, dass** zur Beaufschlagung des Behälters (28, 29) mit Gas eine Druckluftkupplung (36) zur Verbindung mit einer Druckluftquelle, wenigstens eine Behälterkupplung (20, 22) zur Verbindung mit dem Behälter (28, 29) und optional eine Abluftkupplung (38) zur Verbindung mit einer Abluftsenke vorgesehen sind.

4. Prüfvorrichtung zumindest nach Anspruch 3, **dadurch gekennzeichnet, dass** die Behälterkupplungen (20, 22), insbesondere für unterschiedliche oder spezifische Ausprägungen des Behälters (28, 29), unterschiedlich oder spezifisch ausgeprägt sind, so dass sie inkompatibel mit der jeweils anderen spezifischen Ausprägung des Behälters (29, 28) sind, insbesondere nicht mit diesem kuppelbar sind.

5. Prüfvorrichtung nach Anspruch 1, sowie nach Anspruch 3 oder 4, **dadurch gekennzeichnet, dass** die Behälterkupplung (20, 22) einen mit der Steuereinheit signalverbundenen Sensor hat, der ausgestaltet ist, zu erfassen, ob die Behälterkupplung (20, 22) korrekt mit dem Behälter verbunden ist, und/oder dass die Prüfvorrichtung (1; 101) einen mit der Steuereinheit signalverbundenen Sensor hat, der ausgestaltet ist, zu erfassen, ob die Behälterkupplung (20, 22) von einem Steckplatz (16, 18) der Prüfvorrichtung (1; 101), an dem sie vorzugsweise bei Nichtgebrauch vorgehalten ist, entnommen ist oder nicht.

6. Prüfvorrichtung zumindest nach Anspruch 3, **gekennzeichnet durch** einen Druckluftpfad (42), über den die Druckluftkupplung (36) mit der wenigstens einen Behälterkupplung (20, 22) verbindbar ist und einen Abluftpfad (58), über den die wenigstens eine Behälterkupplung (20, 22) mit der Abluftsenke, insbesondere mit Atmosphäre verbindbar ist.

7. Prüfvorrichtung nach Anspruch 6, **dadurch gekennzeichnet, dass** im Druckluftpfad (42) eine erste Filtereinheit (50) zur Unterbindung einer Kontamination des Behälters (28, 29) angeordnet ist, und/oder dass im Abluftpfad (58) eine zweite Filtereinheit (64) zur Unterbindung einer Kontamination der Abluftsenke, insbesondere der Atmosphäre, angeordnet ist.

8. Prüfvorrichtung nach Anspruch 6 oder 7, **gekennzeichnet durch** einen Abzweig (56), an dem der Abluftpfad (58) von dem Druckluftpfad (42) abgezweigt ist, wobei im Druckluftpfad (42) zwischen dem Abzweig (56) und der Druckluftkupplung (36) ein Rückschlagventil (54) angeordnet ist, das in Richtung hin zu der Druckluftkupplung (36) schließt und in Gegenrichtung öffnet.

9. Prüfvorrichtung nach einem der Ansprüche 6 bis 8, **dadurch gekennzeichnet, dass** in dem Druckluftpfad (42) und in dem Abluftpfad (58) jeweils ein betätigbares Sperrventil (52, 62) angeordnet ist, über das der Druckluftpfad (42) und der Abluftpfad (58) unabhängig voneinander, insbesondere über die Steuereinheit, aufsteuerbar und zusteuerbar sind.

10. Prüfvorrichtung nach einem der Ansprüche 6 bis 9, **gekennzeichnet durch** ein Auswahlventil (46) mit einem Versorgungsanschluss (44), der mit dem Druckluftpfad (42) und mit dem Abluftpfad (58), insbesondere mit dem Abzweig (56), verbunden ist, und mit je einem Behälteranschluss pro Behälterkupplung (20, 22), und mit je einer Stellung pro Behälteranschluss, wobei in der jeweiligen Stellung der Versorgungsanschluss (44) mit dem Behälteranschluss verbunden ist und gegen die anderen Behälteranschlüsse gesperrt ist.

11. Prüfvorrichtung nach einem der vorhergehenden Ansprüche, **gekennzeichnet durch** eine, insbesondere mobile, Einhausung (2; 102) mit einer, insbesondere sensorüberwachten, Tür (10; **110), durch** die die Prüfvorrichtung (1; 101) mit dem oder den zu prüfenden Behälter (28, 29) oder Behältern bestückbar ist.

12. Prüfsystem zur Dichtigkeitsprüfung eines medizinischen Behälters, mit einer Prüfvorrichtung (1; 101), die gemäß einem der vorhergehenden Ansprüche ausgestaltet ist, und mit dem medizinischen Behälter (28, 29), welcher vorgesehen und ausgestaltet ist, in ihm eine medizinische Trockensubstanz oder ein medizinisches Trockenkonzentrat in Lösung zu bringen.

13. Verfahren für eine Dichtigkeitsprüfung eines medizinischen Behälters (28, 29) mit einer mit dem Behälter (28, 29) fluidisch verbundenen Prüfvorrichtung (1; 101), die insbesondere gemäß einem der vorhergehenden Ansprüche ausgestaltet ist, wobei der Behälter (28, 29) ausgestaltet ist, dass in ihm eine medizinische Trockensubstanz oder ein medizinisches Trockenkonzentrat in Lösung gebracht wird, mit Schritten
- Beaufschlagen des Behälters (28, 29) mit einem Gas, insbesondere mit Druckluft, mittels der Prüfvorrichtung (1); und
- Erfassen einer Behälterantwort in Abhängigkeit einer Dichtigkeit des Behälters mittels einer Erfassungseinheit (40, wobei die Erfassungseinheit eine Druckerfassungseinheit (40) und die erfasste Behälterantwort ein erfasster Druck des Behälters (28, 29) über die Zeit ist, insbesondere ein Druckabfall des Behälters (28, 29) über die Zeit, **gekennzeichnet durch** einen Schritt
- Abgleichen des erfassten Drucks über die Zeit gegen eine in einer Steuereinheit der Prüfvorrichtung (1; 101) hinterlegte, zeitabhängige Druckkennlinie (+) des Behälters (28, 29) und deren Toleranz (x), mittels der Steuereinheit; und in Abhängigkeit des Abgleichs
- Ausgeben eines Prüfergebnisses "dicht" bei Einhaltung der Toleranz (x) oder "undicht" bei Verletzung der Toleranz (x) oder einer Fehlermeldung oder einer Information, mittels der Steuereinheit an eine mit ihr signalverbundene Bedienschnittstelle der Prüfvorrichtung (1; 101).

14. Verfahren nach Anspruch 13 mit der Prüfvorrichtung (1; 101) gemäß Anspruch 5, insbesondere gemäß Anspruch 4 und 5, **gekennzeichnet durch** vorbereitende Schritte
- Verbinden einer, insbesondere spezifisch ausgeprägten, Behälterkupplung (20, 22) der Prüfvorrichtung (1; 101) mit einer Kupplung des, insbesondere spezifisch ausgeprägten, Behälters (28, 29);
- Auswählen einer Ausprägung des Behälters (28, 29) **durch** einen Bediener an einer Bedienschnittstelle oder automatisiert mittels einer sensorüberwachten, mit einer Steuereinheit der Prüfvorrichtung signalverbundenen Behälterkupplung (20, 22); und
- Sensorüberwachtes oder visuelles Prüfen, ob die Behälterkupplung (20, 22) korrekt mit der Kupplung verbunden ist, und/oder ob die Behälterkupplung (20, 22) mit der Kupplung kompatibel ist; und wenn ja
- Ausgeben einer Bestätigung über die Steuereinheit an die Bedienschnittstelle; und wenn nein
- Ausgeben einer Fehlermeldung über die Steuereinheit an die Bedienschnittstelle und/oder Unterbrechen der Prüfung über die Steuereinheit.

15. Verfahren nach Anspruch 13 oder 14 **gekennzeichnet durch** vorbereitende Schritte:
- Verbinden einer Druckluftkupplung (36) der Prüfvorrichtung (1; 101) mit einer Kupplung einer Druckluftquelle; und
- Absperren oder Schließen aller sonstigen Anschlüsse oder Öffnungen des Behälters (28, 29); und optional
- Verbinden einer Abluftkupplung (38) der Prüfvorrichtung (1; 101) mit einer Kupplung einer Abluftsenke.

16. Verfahren nach Anspruch 15, **gekennzeichnet durch** Schritte:
- Starten der Prüfung mittels einer Bedienschnittstelle;
- Verbinden der Behälterkupplung (20, 22) mit der Druckluftquelle mittels Aufsteuern eines Druckluftpfades (42);
- Trennen der Behälterkupplung (20, 22) von der Druckluftquelle mittels Zusteuern des Druckluftpfades (42) mit Erfassen eines vorbestimmten, behälterspezifischen Start-Drucks; in Folge
- Abgleichen des erfassten Drucks über die Zeit gegen eine hinterlegte, zeitabhängige Druckkennlinie (+) des Behälters (28, 29) und eine Toleranz (x) dieser Druckkennlinie (+); und in Abhängigkeit des Abgleichs, insbesondere in Abhängigkeit einer Einhaltung oder Verletzung der Toleranz (x);
- Ausgeben eines Ergebnisses "dicht" oder "undicht" und/oder einer Fehlermeldung für den Behälter (28, 29).

17. Verfahren nach Anspruch 16 mit einem vorbestimmten, behälterspezifischen Überdruck zum vorbestimmten, behälterspezifischen Start-Druck, **dadurch gekennzeichnet, dass** das Trennen der Behälterkupplung (20, 22) von der Druckluftquelle mit dem Erfassen des vorbestimmten, behälterspezifischen Überdrucks erfolgt.

18. Verfahren nach Anspruch 17 mit einem vorbestimmten, behälterspezifischen Unterdruck zum vorbestimmten, behälterspezifischen Startdruck, **dadurch gekennzeichnet, dass** mit dem Erfassen des vorbestimmten, behälterspezifischen Unterdrucks das erneute Verbinden der Behälterkupplung (20, 22) mit der Druckluftquelle erfolgt, und dass in Folge, mit Erfassen des vorbestimmten, behälterspezifischen Start-Drucks das erneute Trennen der Behälterkupplung von der Druckluftquelle erfolgt.

## Claims

1. A testing device for testing the leak-tightness of a medical container (28, 29), which is provided and configured to bring a medical dry substance or a medical dry concentrate into solution therein, wherein the testing device (1; 101) is provided and configured to apply a gas to the container (28, 29) and to detect a container response dependent on the application and a leak-tightness of the container (28, 29), wherein the container response is a pressure over time, and wherein the testing device (1; 101) has a pressure detection unit (40) for detecting the container response, which is provided and configured for connection to the container (28, 29), **characterized by** a control unit, in which a time-dependent pressure characteristic curve (+) of the container (28, 29) and a tolerance (x) with respect to this pressure characteristic curve (+) are stored, and which is configured to compare the detected pressure over time against the time-dependent pressure characteristic curve (+) for compliance with the tolerance (x) and, depending on the comparison, to output a test result "tight" or "leaky" or an error message or information.

2. The testing device according to claim 1, **characterized in that** the pressure characteristic curve (+) and tolerance (x) are each stored in the control unit for specific embodiments of the container (28, 29).

3. The testing device according to one of the preceding claims, **characterized in that** a compressed air coupling (36) for connection to a compressed air source, at least one container coupling (20, 22) for connection to the container (28, 29) and optionally an exhaust air coupling (38) for connection to an exhaust air sink are provided for applying gas to the container (28, 29).

4. The testing device at least according to claim 3, **characterized in that** the container couplings (20, 22), in particular for different or specific embodiments of the container (28, 29), are designed differently or specifically, so that they are incompatible with the respective other specific design of the container (29, 28), in particular they cannot be coupled thereto.

5. The testing device according to claim 1 as well as according to claim 3 or 4, **characterized in that** the container coupling (20, 22) has a sensor which is signal-connected to the control unit and which is configured to detect whether the container coupling (20, 22) is correctly connected to the container, and/or that the testing device (1; 101) has a sensor which is signal-connected to the control unit and which is configured to detect whether or not the container coupling (20, 22) is removed from a slot (16, 18) of the testing device (1; 101), at which it is preferably kept ready when not in use.

6. The testing device at least according to claim 3, **characterized by** a compressed air path (42), via which the compressed air coupling (36) can be connected to the at least one container coupling (20, 22), and an exhaust air path (58), via which the at least one container coupling (20, 22) can be connected to the exhaust air sink, in particular to the atmosphere.

7. The testing device according to claim 6, **characterized in that** a first filter unit (50) for preventing contamination of the container (28, 29) is arranged in the compressed air path (42), and/or that a second filter unit (64) for preventing contamination of the exhaust air sink, in particular of the atmosphere, is arranged in the exhaust air path (58).

8. The testing device according to claim 6 or 7, **characterized by** a branch (56), at which the exhaust air path (58) is branched off from the compressed air path (42), wherein a non-return valve (54) is arranged in the compressed air path (42) between the branch (56) and the compressed air coupling (36), which closes in the direction towards the compressed air coupling (36) and opens in the opposite direction.

9. The testing device according to one of claims 6 to 8, **characterized in that** an actuatable shut-off valve (52, 62) is arranged in the compressed air path (42) and in the exhaust air path (58) and via which the compressed air path (42) and the exhaust air path (58) can be opened and closed independently of each other, in particular via the control unit.

10. The testing device according to one of claims 6 to 9, **characterized by** a selection valve (46) with a supply connection (44), which is connected to the compressed air path (42) and to the exhaust air path (58), in particular to the branch (56), and with in each case one container connection per container coupling (20, 22), and with in each case one position per container connection, wherein in the respective position, the supply connection (44) is connected to the container connection and is blocked against the other container connections.

11. The testing device according to one of the preceding claims, **characterized by** an, in particular mobile, housing (2; 102) with an, in particular sensor-monitored, door (10; 110), through which the testing device (1; 101) can be equipped with the container or containers (28, 29) to be tested.

12. A testing system for leak testing a medical container, with a testing device (1; 101), which is configured according to one of the preceding claims, and with the medical container (28, 29), which is provided and configured to bring a medical dry substance or a medical dry concentrate into solution therein.

13. A method for leak testing a medical container (28, 29) with a testing device (1; 101), which is fluidically connected to the container (28, 29) and which is configured in particular according to one of the preceding claims, wherein the container (28, 29) is configured such that a medical dry substance or a medical dry concentrate is brought into solution therein, with steps
- applying a gas, in particular compressed air, to the container (28, 29) via the testing device (1); and
- detecting a container response depending on a leak-tightness of the container via a detection unit (40), wherein the detection unit is a pressure detection unit (40) and the detected container response is a detected pressure of the container (28, 29) over time, in particular a pressure drop of the container (28, 29) over time, **characterized by** a step
- comparing the detected pressure over time against a time-dependent pressure characteristic curve (+) of the container (28, 29) and the tolerance (x) thereof stored in a control unit of the testing device (1; 101), via the control unit; and, depending on the comparison,
- outputting a test result "tight" when the tolerance (x) is complied with or "leaky" when the tolerance (x) is violated or an error message or information, via the control unit to an operating interface of the testing device (1; 101), which is signal-connected thereto.

14. The method according to claim 13 with the testing device (1; 101) according to claim 5, in particular according to claims 4 and 5, **characterized by** preparatory steps
- connecting a container coupling (20, 22), in particular specifically designed, of the testing device (1; 101) to a coupling of the container (28, 29), in particular specifically designed;
- selecting a design of the container (28, 29) by an operator at an operating interface or automatically via a sensor-monitored container coupling (20, 22), which is signal-connected to a control unit of the testing device; and
- sensor-monitored or visual testing, whether the container coupling (20, 22) is correctly connected to the coupling, and/or whether the container coupling (20, 22) is compatible with the coupling; and if so
- outputting a confirmation via the control unit to the operating interface; and if not
- outputting an error message via the control unit to the operating interface and/or interrupting the test via the control unit.

15. The method according to claim 13 or 14, **characterized by** preparatory steps:
- connecting a compressed air coupling (36) of the testing device (1; 101) to a coupling of a compressed air source; and
- blocking or closing all other connections or openings of the container (28, 29); and optionally
- connecting an exhaust air coupling (38) of the testing device (1; 101) to a coupling of an exhaust air sink.

16. The method according to claim 15, **characterized by** steps:
- starting the test via an operating interface;
- connecting the container coupling (20, 22) to the compressed air source by opening a compressed air path (42);
- disconnecting the container coupling (20, 22) from the compressed air source by closing the compressed air path (42) while detecting a predetermined, container-specific starting pressure; as a result
- comparing the detected pressure over time against a stored, time-dependent pressure characteristic curve (+) of the container (28, 29) and a tolerance (x) of this pressure characteristic curve (+); and, depending on the comparison, in particular depending on compliance with or violation of the tolerance (x);
- outputting a result "tight" or "leaky" and/or an error message for the container (28, 29).

17. The method according to claim 16 with a predetermined, container-specific overpressure relative to the predetermined, container-specific starting pressure, **characterized in that** the container coupling (20, 22) is disconnected from the compressed air source while detecting the predetermined, container-specific overpressure.

18. The method according to claim 17 with a predetermined, container-specific underpressure relative to the predetermined, container-specific starting pressure, **characterized in that** the container coupling (20, 22) is again connected to the compressed air source while detecting the predetermined, container-specific underpressure, and that as a result, the container coupling is again disconnected from the compressed air source while detecting the predetermined, container-specific starting pressure.

## Revendications

1. Dispositif de contrôle pour le contrôle d'étanchéité d'un récipient médical (28, 29) qui est prévu et conçu pour y mettre en solution une substance médicale sèche ou un concentré médical sec, dans lequel le dispositif de contrôle (1; 101) est prévu et conçu pour alimenter le récipient (28, 29) en un gaz et pour détecter une réponse de récipient dépendante de l'alimentation et de l'étanchéité du récipient (28, 29), dans lequel la réponse de récipient est une pression dans le temps, et dans lequel le dispositif de contrôle (1; 101) présente une unité de détection de pression (40) pour détecter la réponse de récipient, unité qui est prévue et conçue pour la connexion au récipient (28, 29), **caractérisé par** un dispositif de commande dans lequel une courbe caractéristique de pression (+) en fonction du temps du récipient (28, 29) et une tolérance (x) par rapport à cette courbe caractéristique de pression (+) sont enregistrées, et qui est conçue pour comparer la pression détectée dans le temps à la courbe caractéristique de pression (+) en fonction du temps par rapport à un respect de la tolérance (x) et, en fonction de la comparaison, pour émettre un résultat de contrôle « étanche » ou « non étanche » ou un message d'erreur ou une information.

2. Dispositif de contrôle selon la revendication 1, **caractérisé en ce que** la courbe caractéristique de pression (+) et la tolérance (x) sont respectivement enregistrées dans le dispositif de commande pour des mises en œuvre spécifiques du récipient (28, 29).

3. Dispositif de contrôle selon l'une quelconque des revendications précédentes, **caractérisé en ce que**, pour alimenter le récipient (28, 29) en gaz, un accouplement à air comprimé (36) est prévu pour la connexion à une source d'air comprimé, au moins un accouplement de récipient (20, 22) pour la connexion au récipient (28, 29) et facultativement un accouplement d'air d'évacuation (38) pour la connexion à un puits d'air d'évacuation.

4. Dispositif de contrôle selon au moins la revendication 3, **caractérisé en ce que** les accouplements de récipient (20, 22), en particulier pour des mises en œuvre différentes ou spécifiques du récipient (28, 29), sont mis en œuvre de manière différente ou spécifique, de sorte qu'ils sont incompatibles respectivement avec l'autre mise en œuvre spécifique du récipient (29, 28), en particulier ne peuvent pas être couplés à celui-ci.

5. Dispositif de contrôle selon la revendication 1, ainsi que selon la revendication 3 ou 4, **caractérisé en ce que** l'accouplement de récipient (20, 22) présente un capteur connecté par signal au dispositif de commande, capteur qui est conçu pour détecter si l'accouplement de récipient (20, 22) est correctement connecté au récipient, et/ou **en ce que** le dispositif de contrôle (1 ; 101) présente un capteur connecté par signal au dispositif de commande, capteur qui est conçu pour détecter si l'accouplement de récipient (20, 22) a été retiré ou non d'un logement (16, 18) du dispositif de contrôle (1 ; 101), au niveau duquel il est de préférence conservé lorsqu'il n'est pas utilisé.

6. Dispositif de contrôle selon au moins la revendication 3, **caractérisé par** un circuit d'air comprimé (42) par l'intermédiaire duquel l'accouplement à air comprimé (36) peut être connecté à l'au moins un accouplement de récipient (20, 22) et un circuit d'air évacué (58) par l'intermédiaire duquel le au moins un accouplement de récipient (20, 22) peut être connecté au puits d'air évacué, en particulier à l'atmosphère.

7. Dispositif de contrôle selon la revendication 6, **caractérisé en ce qu'**une première unité de filtration (50) est disposée dans le circuit d'air comprimé (42) pour empêcher une contamination du récipient (28, 29), et/ou **en ce qu'**une seconde unité de filtration (64) est disposée dans le circuit d'air évacué (58) pour empêcher une contamination du puits d'air évacué, en particulier de l'atmosphère.

8. Dispositif de contrôle selon la revendication 6 ou 7, **caractérisé par** une dérivation (56) au niveau de laquelle le circuit d'air évacué (58) est dérivé du circuit d'air comprimé (42), dans lequel un clapet de retenue (54) est disposé dans le circuit d'air comprimé (42) entre la dérivation (56) et l'accouplement d'air comprimé (36), clapet qui se ferme en direction de l'accouplement d'air comprimé (36) et s'ouvre dans la direction opposée.

9. Dispositif de contrôle selon l'une quelconque des revendications 6 à 8, **caractérisé en ce qu'**une vanne d'arrêt actionnable (52, 62) est disposée respectivement dans le circuit d'air comprimé (42) et dans le circuit d'air évacué (58), vanne par l'intermédiaire de laquelle le circuit d'air comprimé (42) et le circuit d'air évacué (58) peuvent être commandés et régulés indépendamment l'un de l'autre, en particulier par l'intermédiaire du dispositif de commande.

10. Dispositif de contrôle selon l'une quelconque des revendications 6 à 9, **caractérisé par** une vanne de sélection (46) avec un raccord d'alimentation (44) qui est connecté au circuit d'air comprimé (42) et au circuit d'air évacué (58), en particulier à la dérivation (56), et chacun avec un raccord de récipient par accouplement de récipient (20, 22) et chacun avec une position par raccord de récipient, dans lequel le raccord d'alimentation (44) est connecté à l'accouplement de récipient dans la position respective et est bloqué par rapport aux autres raccords de récipient.

11. Dispositif de contrôle selon l'une quelconque des revendications précédentes, **caractérisé par** une enceinte (2 ; 102), en particulier mobile, avec une porte (10 ; 110), en particulier surveillée par capteur, à travers laquelle le dispositif de contrôle (1 ; 101) peut être équipé du ou des récipients (28, 29) à contrôler.

12. Système de contrôle pour le contrôle d'étanchéité d'un récipient médical, avec un dispositif de contrôle (1 ; 101) qui est conçu selon l'une quelconque des revendications précédentes, et avec le récipient médical (28, 29) qui est prévu et conçu pour y mettre en solution une substance médicale sèche ou un concentré médical sec.

13. Procédé de contrôle d'étanchéité d'un récipient médical (28, 29) avec un dispositif de contrôle (1 ; 101) connecté de manière fluidique au récipient (28, 29), dispositif qui est en particulier conçu selon l'une quelconque des revendications précédentes, dans lequel le récipient (28, 29) est conçu pour y mettre en solution une substance médicale sèche ou un concentré médical sec, avec les étapes de
- alimentation du récipient (28, 29) en un gaz, en particulier en air comprimé, au moyen du dispositif de contrôle (1) ; et
- détection d'une réponse de récipient en fonction d'une étanchéité du récipient au moyen d'une unité de détection (40), dans lequel l'unité de détection est une unité de détection de pression (40), et la réponse de récipient détectée est une pression détectée du récipient (28, 29) dans le temps, en particulier une chute de pression du récipient (28, 29) dans le temps, **caractérisé par** une étape de
- comparaison de la pression détectée dans le temps avec une courbe caractéristique de pression (+) du récipient (28, 29) en fonction du temps et enregistrée dans un dispositif de commande du dispositif de contrôle (1 ; 101) et sa tolérance (x), au moyen du dispositif de commande ; et en fonction de la comparaison
- émission d'un résultat de contrôle « étanche » en cas de respect de la tolérance (x) ou « non étanche » en cas de non-respect de la tolérance (x) ou d'un message d'erreur ou d'une information, au moyen du dispositif de commande, au niveau d'une interface utilisateur du dispositif de contrôle (1 ; 101) connectée par signal à celle-ci.

14. Procédé selon la revendication 13 avec le dispositif de contrôle (1 ; 101) selon la revendication 5, en particulier selon les revendications 4 et 5, **caractérisé par** des étapes préparatoires de
- connexion d'un accouplement de récipient (20, 22), en particulier de mise en œuvre spécifique, du dispositif de contrôle (1 ; 101) à un accouplement du récipient (28, 29), en particulier de mise en œuvre spécifique ;
- sélection d'une mise en œuvre du récipient (28, 29) par un opérateur sur une interface utilisateur ou de manière automatisée au moyen d'un accouplement de récipient (20, 22) surveillé par capteur, connecté par signal à un dispositif de commande du dispositif de contrôle ; et
- contrôle surveillé par capteur ou visuel établissant si l'accouplement de récipient (20, 22) est correctement connecté à l'accouplement et/ou si l'accouplement de récipient (20, 22) est compatible avec l'accouplement ; et si oui
- émission d'une confirmation par l'intermédiaire du dispositif de commande au niveau de l'interface utilisateur ; et si non
- émission d'un message d'erreur par l'intermédiaire du dispositif de commande au niveau de l'interface utilisateur et/ou interruption du contrôle par l'intermédiaire du dispositif de commande.

15. Procédé selon la revendication 13 ou 14, **caractérisé par** des étapes préparatoires de :
- connexion d'un accouplement à air comprimé (36) du dispositif de contrôle (1 ; 101) à un accouplement d'une source d'air comprimé ; et
- fermeture ou blocage de tous les autres raccords ou ouvertures du récipient (28, 29) ; et facultativement
- connexion d'un accouplement d'air évacué (38) du dispositif de contrôle (1 ; 101) à un accouplement d'un puits d'air évacué.

16. Procédé selon la revendication 15, **caractérisé par** des étapes de :
- lancement du contrôle au moyen d'une interface utilisateur ;
- connexion de l'accouplement de récipient (20, 22) à la source d'air comprimé au moyen d'une commande d'un circuit d'air comprimé (42) ;
- séparation de l'accouplement de récipient (20, 22) de la source d'air comprimé au moyen d'une commande du circuit d'air comprimé (42) avec détection d'une pression de départ prédéterminée spécifique au récipient ; puis
- comparaison de la pression détectée dans le temps avec une courbe caractéristique de pression (+) enregistrée et dépendante du temps du récipient (28, 29) et une tolérance (x) de cette courbe caractéristique de pression (+) ; et en fonction de la comparaison, en particulier en fonction d'un respect ou d'un non-respect de la tolérance (x) ;
- émission d'un résultat « étanche » ou « non étanche » et/ou d'un message d'erreur pour le récipient (28, 29).

17. Procédé selon la revendication 16 avec une surpression prédéterminée
spécifique au récipient par rapport à la pression de départ prédéterminée spécifique au récipient, **caractérisé en ce que** la séparation de l'accouplement de récipient (20, 22) de la source d'air comprimé est effectuée avec la détection de la surpression prédéterminée spécifique au récipient.

18. Procédé selon la revendication 17 avec une dépression prédéterminée
spécifique au récipient par rapport à la pression de départ prédéterminée spécifique au récipient, **caractérisé en ce que**, lorsque la dépression prédéterminée
spécifique au récipient est détectée, la connexion renouvelée de l'accouplement de récipient (20, 22) à la source d'air comprimé est effectuée, et **en ce que**, avec la détection de la pression de départ prédéterminée spécifique au récipient, la séparation renouvelée de l'accouplement de récipient de la source d'air comprimé est effectuée.
